(19) Europäisches Patentamt
European Patent Office
Office européen des brevets

(11) EP 4 749 629 A1

(12) EUROPEAN PATENT APPLICATION
published in accordance with Art. 153(4) EPC

(43) Date of publication:
27.05.2026 Bulletin 2026/22

(21) Application number: 24851721.1

(22) Date of filing: 31.07.2024

(51) International Patent Classification (IPC):
*G16C 20/50* (2019.01)

(52) Cooperative Patent Classification (CPC):
G16C 20/50

(86) International application number:
PCT/JP2024/027412

(87) International publication number:
WO 2025/033296 (13.02.2025 Gazette 2025/07)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 10.08.2023 JP 2023131649

(71) Applicant: CHUGAI SEIYAKU KABUSHIKI KAISHA
Tokyo 115-8543 (JP)

(72) Inventors:
- TAKIZAWA Shuuki
Yokohama City, Kanagawa 244-8602 (JP)
- MORI Keita
Yokohama City, Kanagawa 244-8602 (JP)
- YOSHIMURA Dai
Yokohama City, Kanagawa 244-8602 (JP)
- TERAMOTO Reiji
Yokohama City, Kanagawa 244-8602 (JP)

(74) Representative: Vossius & Partner
Patentanwälte Rechtsanwälte mbB
Siebertstraße 3
81675 München (DE)

# (54) INFORMATION PROCESSING SYSTEM, INFORMATION PROCESSING METHOD, PROGRAM, AND METHOD FOR MANUFACTURING MOLECULAR COMPOUND

(57) An information processing system for identifying a molecule suitable for a drug candidate comprises a characteristic predictor configured to calculate, with respect to a molecule forming an element of a sequence information collection which is a collection of sequence information on a plurality of different molecules, a characteristic predicted value of the molecule using a prediction model for predicting a characteristic of a molecule from sequence information on the molecule, followed by estimation of uncertainty of the prediction, and a candidate molecule identifier configured to search for a candidate of a molecule having a desired characteristic on the basis of the characteristic predicted value and an estimate of the uncertainty of prediction.

*Fig.1*

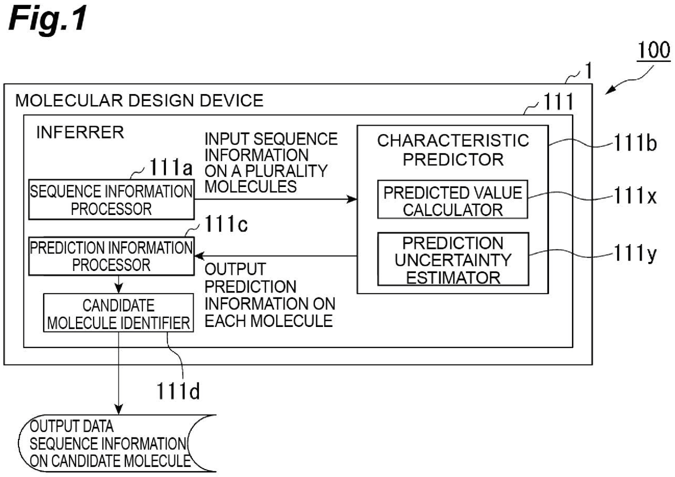


EP 4 749 629 A1

## Description

[Technical Field]

An aspect of the present disclosure relates to an information processing system, a molecular design device, an information processing method, a program, and a method for producing a molecular compound.

[Background Art]

Recently, in the field of pharmaceuticals, information processing techniques of machine learning have been utilized for reducing the burden in drug discovery (Patent Literature 1). Attempts have been made to utilize prediction by machine learning to efficiently discover a molecule having a properties required for a drug.

[Citation List]

[Patent Literature]

[Patent Literature 1] International Publication No. WO 2018/132752

[Summary of Invention]

[Technical Problem]

Prediction by machine learning entails uncertainty, and failure to take this into consideration for utilizing prediction by machine learning may lead to a result that does not match expectations. In drug discovery, there are many targets to be analyzed, and it is difficult to apply prediction by machine learning to all of targets on which the prediction by machine learning can be performed.

In view of the above-described circumstances, an object of an aspect of the present disclosure is to provide a technique for further reducing the burden accompanying drug discovery.

[Solution to Problem]

An aspect of the present disclosure provides the following modes.

[A1]
An information processing system for identifying a molecule suitable for a drug candidate, comprising:

- a characteristic predictor configured to calculate, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
- a candidate molecule identifier configured to search for a candidate of a molecule having a desired characteristic on the basis of the characteristic predicted value and an estimate of the uncertainty of prediction.

[A2]
The information processing system according to A1, wherein the building block combination information is sequence information on the molecule.
[A3]
The information processing system according to A1 or A2, wherein the molecule is at least one of a nucleic acid, a peptide, a cyclic peptide, a protein, an antibody, and a low-molecular compound.
[A4]
The information processing system according to any one of A1 to A3, wherein the molecule is a protein, an antibody, a peptide or a cyclic peptide, and the building block combination information is information on amino acid sequences.
[A5]
The information processing system according to any one of A1 to A4, wherein the characteristic is at least one of binding ability, pharmacological activity, a physical property, kinetics, and safety.
[A6]

The information processing system according to any one of A1 to A5, wherein the molecule is a molecule that binds to a target molecule, and the characteristic is ability to bind to the target molecule.

[A7]

The information processing system according to any one of A1 to A6, further comprising a prediction information processor configured to calculate a characteristic quality value on the basis of the characteristic predicted value and the estimate of uncertainty of prediction,

wherein the candidate molecule identifier identifies at least one candidate molecule from elements of the building block combination information collection on the basis of the characteristic quality value.

[A8]

The information processing system according to any one of A1 to A7, wherein the characteristic quality value increases with an increase in the characteristic predicted value, and decreases with a decrease in the estimate of uncertainty of prediction.

[A9]

The information processing system according to A8, wherein the characteristic quality value is an output value of a predetermined function in which two variables, one of which is the characteristic predicted value and the other of which is the estimate of uncertainty of prediction, are inputs.

[A10]

The information processing system according to any one of A7 to A9, wherein the prediction information processor calculates a mean variance as an objective function that gives the characteristic quality value.

[A11]

The information processing system according to any one of A1 to A10, wherein the estimate of uncertainty of prediction is a standard deviation of the characteristic predicted value.

[A12]

The information processing system according to any one of A1 to A11, wherein the characteristic quality value increases with an increase in the characteristic predicted value, and decreases with a decrease in the estimate of uncertainty of prediction, the building block combination information collection is acquired using a combination optimization algorithm, and an extraction parameter of the combination optimization algorithm is updated on the basis of the characteristic predicted value of the molecule or the characteristic quality value.

[A13]

The information processing system according to A12, wherein the combination optimization algorithm is a tree-structured Parzen estimator.

[A14]

The information processing system according to any one of A1 to A13, wherein the prediction model is a prediction model generated by learning based on building block combination information on a plurality of molecules for training and results of evaluating the characteristics of the molecules.

[B1]

A molecular design device comprising a controller configured to infer a characteristic of a molecule from building block combination information on the molecule using a predetermined prediction model,

wherein the controller

acquires a building block combination information collection which is a collection of building block combination information on a plurality of different molecules,

calculates, with respect to a molecule forming an element of the building block combination information collection, a characteristic predicted value of the molecule from the building block combination information on the molecule with the prediction model, estimates uncertainty of the prediction, and

searches for a candidate of a molecule having a desired characteristic on the basis of the characteristic predicted value and an estimate of the uncertainty of prediction.

[B2]

The molecular design device according to B1, wherein the building block combination information is sequence information on the molecule.

[B3]

The molecular design device according to B1 or B2, wherein the molecule is at least one of a nucleic acid, a peptide, a cyclic peptide, a protein, an antibody, and a low-molecular compound.

[B4]

The molecular design device according to any one of B1 to B3, wherein the molecule is a protein, an antibody, a peptide or a cyclic peptide, and the building block combination information is information on amino acid sequences.

[B5]

The molecular design device according to any one of B1 to B4, wherein the characteristic is at least one of binding ability, pharmacological activity, a physical property, kinetics, and safety.

[B6]

The molecular design device according to any one of B1 to B5, wherein the molecule is a molecule that binds to a target molecule, and the characteristic is ability to bind to the target molecule.

[B7]

The molecular design device according to any one of B1 to B6, wherein the controller further calculates a characteristic quality value on the basis of the characteristic predicted value and the estimate of uncertainty of prediction, and identifies at least one candidate molecule from elements of the building block combination information collection on the basis of the characteristic quality value.

[B8]

The molecular design device according to B7, wherein the characteristic quality value increases with an increase in the characteristic predicted value, and decreases with a decrease in the estimate of uncertainty of prediction.

[B9]

The molecular design device according to B8, wherein the characteristic quality value is an output value of a predetermined function in which two variables, one of which is the characteristic predicted value and the other of which is the estimate of uncertainty of prediction, are inputs.

[B10]

The molecular design device according to any one of B7 to B9, wherein the controller calculates a mean variance as an objective function that gives the characteristic quality value.

[B11]

The molecular design device according to any one of B1 to B10, wherein the estimate of uncertainty of prediction is a standard deviation of the characteristic predicted value.

[B12]

The molecular design device according to any one of B1 to B11, wherein the controller acquires the building block combination information collection using a combination optimization algorithm, and updates an extraction parameter of the combination optimization algorithm on the basis of the characteristic predicted value or the characteristic quality value.

[B13]

The molecular design device according to B12, wherein the combination optimization algorithm is a tree-structured Parzen estimator.

[B14]

The molecular design device according to any one of B1 to B13, further comprising an output configured to output building block combination information on the candidate molecule.

[B15]

The molecular design device according to any one of B1 to B14, wherein the prediction model is a prediction model generated by learning based on building block combination information on a plurality of molecules for training and results of evaluating the characteristics of the molecules.

[C1]

An information processing system for identifying a molecule suitable for a drug candidate, comprising:

a building block combination information processor configured to acquire, in accordance with a combination optimization algorithm, a building block combination information collection which is a collection of building block combination information on a plurality of different molecules;
a characteristic predictor configured to calculate, with respect to a molecule forming an element of the building block combination information collection, a characteristic predicted value using a prediction model for predicting a characteristic of a molecule from building block combination information on the molecule; and
a candidate molecule identifier configured to search for a candidate of a molecule having a desired characteristic on the basis of the characteristic predicted value,
wherein the building block combination information processor
updates an extraction parameter of the combination optimization algorithm on the basis of the characteristic predicted value of the molecule so as to include a molecule having a more desired characteristic.

[C2]

The information processing system according to C1, wherein the candidate molecule identifier searches for a candidate of a molecule having a desired characteristic on the basis of a first characteristic predicted value calculated with respect to a molecule forming an element of a first building block combination information collection which is a building block combination information collection acquired before the extraction parameter is updated, and a second characteristic predicted value calculated with respect to a molecule forming an element of a second building block combination information collection which is a building block combination information collection acquired after the extraction parameter is updated.

[C3]

The information processing system according to C2, wherein the first building block combination information collection and the second building block combination information collection are collections including building block combination information on different molecules.

[C4]

The information processing system according to any one of C1 to C3, wherein the building block combination information is sequence information on the molecule.

[C5]

The information processing system according to any one of C1 to C4, wherein the molecule is at least one of a nucleic acid, a peptide, a cyclic peptide, a protein, an antibody, and a low-molecular compound.

[C6]

The information processing system according to any one of C1 to C5, wherein the molecule is a protein, an antibody, a peptide or a cyclic peptide, and the building block combination information is information of amino acid sequences.

[C7]

The information processing system according to any one of C1 to C6, wherein the characteristic is at least one of binding ability, pharmacological activity, a physical property, kinetics, and safety.

[C8]

The information processing system according to any one of C1 to C7, wherein the molecule is a molecule that binds to a target molecule, and the characteristic is ability to bind to the target molecule.

[C9]

The information processing system according to any one of C1 to C8, wherein the characteristic predictor calculates the characteristic predicted value, and calculates the estimate of uncertainty of prediction.

[C10]

The information processing system according to any one of C1 to C9, wherein the building block combination information processor

updates an extraction parameter of the combination optimization algorithm on the basis of the characteristic predicted value of the molecule and the estimate of uncertainty of prediction so as to include a molecule having a more desired characteristic.

[C11]

The information processing system according to C9 or C10, further comprising a prediction information processor configured to process prediction information which includes the characteristic predicted value and/or the estimate of uncertainty of prediction, and is output from the characteristic predictor,

wherein the prediction information processor calculates a characteristic quality value on the basis of the characteristic predicted value and the estimate of uncertainty of prediction, and

the candidate molecule identifier identifies at least one molecule from the building block combination information collection on the basis of the characteristic quality value.

[C12]

The information processing system according to C11, wherein the characteristic quality value increases with an increase in the characteristic predicted value, and decreases with a decrease in the estimate of uncertainty of prediction.

[C13]

The information processing system according to C12, wherein the characteristic quality value is an output value of a predetermined function in which two variables, one of which is the characteristic predicted value and the other of which is the estimate of uncertainty of prediction, are inputs.

[C14]

The information processing system according to any one of C11 to C13, wherein the prediction information processor calculates a mean variance as an objective function that gives the characteristic quality value.

[C15]

The information processing system according to any one of C9 to C14, wherein the estimate of uncertainty of prediction is a standard deviation of the characteristic predicted value.

[C16]

The information processing system according to any one of C1 to C15, wherein a tree-structured Parzen estimator is used as the combination optimization algorithm.
[D1]

A molecular design device comprising a controller configured to infer a characteristic of a molecule from building block combination information on the molecule using a predetermined prediction model,
wherein the controller
acquires, in accordance with a combination optimization algorithm, a building block combination information collection which is a collection of building block combination information on a plurality of different molecules,
calculates, using the prediction model, the characteristic predicted value with respect to a molecule forming an element of the building block combination information collection,
updates an extraction parameter of the combination optimization algorithm on the basis of a characteristic predicted value of each molecule with the prediction model so as to include a molecule having a more desired characteristic, and
searches for a candidate of a molecule having a desired characteristic on the basis of the characteristic predicted value.

[D2]

The molecular design device according to D1, wherein the controller
acquires a first building block combination information collection which is a collection of building block combination information on a plurality of different molecules before the extraction parameter is updated,
calculates, with respect to a molecule forming an element of the first building block combination information collection, a first characteristic predicted value using the prediction model,
further acquires a second building block combination information collection which is a collection of building block combination information on a plurality of different molecules after the extraction parameter is updated,
calculates, with respect to a molecule forming an element of the second building block combination information collection, a second characteristic predicted value using the prediction model, and
searches for a candidate of a molecule having a desired characteristic on the basis of the first characteristic predicted value and the second characteristic predicted value.

[D3]
The molecular design device according to D2, wherein the first building block combination information collection and the second building block combination information collection are collections including sequence information on different molecules.
[D4]
The molecular design device according to any one of D1 to D3, wherein the building block combination information is sequence information on the molecule.
[D5]
The molecular design device according to any one of D1 to D5, wherein the molecule is at least one of a nucleic acid, a peptide, a cyclic peptide, a protein, an antibody, and a low-molecular compound.
[D6]
The molecular design device according to any one of D1 to D5, wherein the molecule is a protein, an antibody, a peptide or a cyclic peptide, and the building block combination information is information on amino acid sequences.
[D7]
The molecular design device according to any one of D1 to D6, wherein the characteristic is at least one of binding ability, pharmacological activity, a physical property, kinetics, and safety.
[D8]
The molecular design device according to any one of D1 to D7, wherein the molecule is a molecule that binds to a target molecule, and the characteristic is ability to bind to the target molecule.
[D9]
The molecular design device according to any one of D1 to D8, wherein the controller calculates the characteristic predicted value, and calculates an estimate of uncertainty of prediction.
[D10]
The molecular design device according to any one of D1 to D9, wherein the controller acquires the building block combination information collection on the basis of the characteristic predicted value of the molecule and the estimate of uncertainty of prediction so as to include a molecule having a more desired characteristic.
[D11]

The molecular design device according to D10, wherein the controller further calculates a characteristic quality value on the basis of the characteristic predicted value and the estimate of uncertainty of prediction, and
the candidate molecule identifier identifies at least one molecule from the building block combination information collection on the basis of the characteristic quality value.

[D12]
The molecular design device according to D11, wherein the characteristic quality value increases with an increase in the characteristic predicted value, and decreases with a decrease in the estimate of uncertainty of prediction.
[D13]
The molecular design device according to D12, wherein the characteristic quality value is an output value of a predetermined function in which two variables, one of which is the characteristic predicted value and the other of which is the estimate of uncertainty of prediction, are inputs.
[D14]
The molecular design device according to any one of D11 to D13, wherein the controller calculates a mean variance as an objective function that gives the characteristic quality value.
[D15]
The molecular design device according to any one of D9 to D14, wherein the estimate of uncertainty of prediction is a standard deviation of the characteristic predicted value.
[D16]
The molecular design device according to any one of D1 to D15, wherein a tree-structured Parzen estimator is used as the combination optimization algorithm.
[D17]
The molecular design device according to any one of D1 to D16, wherein the prediction model is a model learned on the basis of training data representing building block combination information different depending on molecules, and characteristics of the molecules.
[E1]

An information processing method in an information processing system for identifying a molecule suitable for a drug candidate, the method comprising the steps of: calculating, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
searching for a candidate of a molecule having a more desired characteristic on the basis of the characteristic predicted value and the uncertainty of prediction.

[E2]
The information processing method according to E1, wherein the building block combination information is information on a sequence of the molecules.
[E3]
The information processing method according to E1 or E2, wherein the molecule is at least one of a nucleic acid, a peptide, a cyclic peptide, a protein, an antibody, and a low-molecular compound.
[E4]
The information processing method according to any one of E1 to E3, wherein the molecule is a protein, an antibody, a peptide or a cyclic peptide, and the building block combination information is information on amino acid sequences.
[E5]
The information processing method according to any one of E1 to E4, wherein the characteristic is at least one of binding ability, pharmacological activity, a physical property, kinetics, and safety.
[E6]
The information processing method according to any one of E1 to E5, wherein the molecule is a molecule that binds to a target molecule, and the characteristic is ability to bind to the target molecule.
[E7]

The information processing method according to any one of E1 to E6, further comprising the step of calculating a characteristic quality value on the basis of the characteristic predicted value and the estimate of uncertainty of prediction,
wherein the step of searching comprises the step of identifying at least one molecule from the building block combination information collection on the basis of the characteristic quality value.

[E8]
The information processing method according to E7, wherein the characteristic quality value increases with an increase in the characteristic predicted value, and decreases with a decrease in the estimate of uncertainty of prediction.
[E9]
The information processing method according to E8, wherein the characteristic quality value is an output value of an arbitrary function in which two variables, one of which is the characteristic predicted value and the other of which is the estimate of uncertainty of prediction, are inputs.
[E10]
The information processing method according to any one of E7 to E9, wherein the step of calculating a characteristic quality value comprises calculating a mean variance as an objective function that gives the characteristic quality value.
[E11]
The information processing method according to any one of E1 to E10, wherein the estimate of uncertainty of prediction is a standard deviation of the predicted value.
[E12]
The information processing method according to any one of E1 to E11, comprising the step of outputting building block combination information on the identified candidate molecule.
[E13]
The information processing method according to E12, comprising further outputting information on a characteristic predicted value of the identified molecule.
[E14]
The information processing method according to any one of E7 to E13, wherein the step of searching comprises the step of identifying a molecule, in which the characteristic quality value is equal to or larger than a predetermined value, from the building block combination information collection.
[E15]
The information processing method according to any one of E7 to E13, wherein the step of searching comprises the step of identifying a rank of the characteristic quality value of each molecule, and identifying a molecule that is at or above a predetermined rank.
[E16]
The information processing method according to any one E1 to E15, wherein the step of searching comprises the step of selecting at least one molecule, in which the characteristic predicted value and the uncertainty of prediction meet predetermined conditions, respectively, from the building block combination information collection.
[E17]
The information processing method according to any one of E1 to E16, comprising acquiring the building block combination information collection using a combination optimization algorithm, and updating an extraction parameter of the combination optimization algorithm on the basis of the characteristic predicted value or the characteristic quality value.
[E18]
The information processing method according to E17, wherein the combination optimization algorithm is a tree-structured Parzen estimator.
[E19]
The information processing method according to any one of E1 to E18, wherein the prediction model is a model learned on the basis of training data representing building block combination information different depending on molecules, and characteristics of the molecules.
[F1]
A program configured to cause a computer to execute:

a procedure of calculating, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
a procedure of searching for a candidate of a molecule having a more desired characteristic on the basis of the characteristic predicted value and the uncertainty of prediction.

[F2]
The program according to F1, wherein the building block combination information is sequence information on the molecule.

[F3]

The program according to F1 or F2, wherein the molecule is at least one of a nucleic acid, a peptide, a cyclic peptide, a protein, an antibody, and a low-molecular compound.

[F4]

The program according to any one of F1 to F3, wherein the molecule is a protein, an antibody, a peptide or a cyclic peptide, and the building block combination information is information on amino acid sequences.

[F5]

The program according to any one of F1 to F4, wherein the characteristic is at least one of binding ability, pharmacological activity, a physical property, kinetics, and safety.

[F6]

The program according to any one of F1 to F5, wherein the molecule is a molecule that binds to a target molecule, and the characteristic is ability to bind to the target molecule.

[F7]

The program according to any one of F1 to F6, further comprising a procedure of calculating a characteristic quality value on the basis of the characteristic predicted value and the estimate of uncertainty of prediction, wherein the procedure of searching comprises a procedure of identifying at least one molecule from the building block combination information collection on the basis of the characteristic quality value.

[F8]

The program according to F7, wherein the characteristic quality value increases with an increase in the characteristic predicted value, and decreases with a decrease in the estimate of uncertainty of prediction.

[F9]

The program according to F8, wherein the characteristic quality value is an output value of a predetermined function in which two variables, one of which is the characteristic predicted value and the other of which is the estimate of uncertainty of prediction, are inputs.

[F10]

The program according to any one of F7 to F9, wherein the procedure of calculating a characteristic quality value comprises calculating a mean variance as an objective function that gives the characteristic quality value.

[F11]

The program according to any one of F1 to F10, wherein the estimate of uncertainty is a standard deviation of the predicted value.

[F12]

The program according to any one of F1 to F11, comprising a procedure of outputting building block combination information on a candidate molecule identified by searching for a candidate of a molecule having a more desired characteristic.

[F13]

The program according to F12, comprising further outputting information on the characteristic predicted value of the candidate molecule identified by searching for a candidate of a molecule having a more desired characteristic.

[F14]

The program according to any one of F7 to F13, wherein the procedure of searching comprises a procedure of identifying a molecule, in which the characteristic quality value is equal to or larger than a predetermined value, from the building block combination information collection.

[F15]

The program according to any one of F7 to F13, wherein the procedure of searching comprises a procedure of identifying a rank of the characteristic quality value of each molecule, and identifying a molecule that is at or above a predetermined rank.

[F16]

The program according to any one of F1 to F15, wherein the procedure of searching comprises a procedure of selecting at least one molecule, in which the characteristic predicted value and the estimate of uncertainty of prediction meet predetermined conditions, respectively, from the building block combination information collection.

[F17]

The program according to any one of F1 to F16, further comprising a procedure of updating an extraction parameter for acquisition of the building block combination information collection on the basis of the characteristic predicted value or the characteristic quality value using a combination optimization algorithm.

[F18]

The program according to F17, wherein the combination optimization algorithm is a tree-structured Parzen estimator.

[F19]
The program according to any one of F1 to F18, wherein the prediction model is a model learned on the basis of training data representing building block combination information different depending on molecules, and characteristics of the molecules.
[G1]
A method for producing a molecular compound, the method comprising:

an input step of accessing a building block combination information collection which is a collection of building block combination information on a plurality of different molecules, and
inputting the building block combination information collection to a prediction model;
an inference step of searching for a molecule having a more desired characteristic from the building block combination collection, on the basis of a characteristic predicted value and an estimate of uncertainty of prediction for each molecule which are contained in the building block combination information collection and output from the prediction model, followed by identification as a candidate molecule;
an output step of outputting building block combination information on the candidate molecule; and
a generation step of generating the molecular compound having a molecular sequence represented by the building block combination information.

[G2]
The method according to G1, wherein the candidate molecule has a biological sequence, and the building block combination information is sequence information.
[G3]
The method according to G1 or G2, wherein the molecule is at least one of a nucleic acid, a peptide, a cyclic peptide, a protein, an antibody, and a low-molecular compound.
[G4]
The method according to any one of G1 to G3, wherein the molecule is a protein, an antibody, a peptide or a cyclic peptide, and the building block combination information is information on amino acid sequences.
[G5]
The method according to any one of G1 to G4, wherein the characteristic is at least one of binding ability, pharmacological activity, a physical property, kinetics, and safety.
[G6]
The method according to any one of G1 to G5, wherein the molecule is a molecule that binds to a target molecule, and the characteristic is ability to bind to the target molecule.
[G7]
The method according to any one of G1 to G6, comprising the step of calculating a characteristic quality value on the basis of the characteristic predicted value and the estimate of uncertainty of prediction, and identifying at least one candidate molecule from the building block combination information collection on the basis of the characteristic quality value.
[G8]
The method according to G7, wherein the characteristic quality value increases with an increase in the characteristic predicted value, and decreases with a decrease in the estimate of uncertainty of prediction.
[G9]
The method according to G8, wherein the characteristic quality value is an output value of a predetermined function in which two variables, one of which is the characteristic predicted value and the other of which is the estimate of uncertainty of prediction, are inputs.
[G10]
The method according to any one of G7 to G9, wherein the step of calculating a characteristic quality value comprises calculating a mean variance as an objective function that gives the characteristic quality value.
[G11]
The method according to any one of G1 to G10, wherein the estimate of uncertainty is a standard deviation of the predicted value.
[G12]
The method according to any one of G1 to G11, wherein the information on the candidate molecule includes building block combination information on the candidate molecule.
[G13]
The method according to any one of G1 to G12, wherein the information on the candidate molecule includes a characteristic predicted value of the candidate molecule.
[G14]

The method according to any one of G7 to G13, wherein the step of searching for a candidate molecule comprises the step of identifying, as the candidate molecule, a molecule, in which the characteristic quality value is equal to or larger than a predetermined value, from the building block combination information collection.
[G15]
The method according to any one of G7 to G13, wherein the step of searching for a candidate molecule comprises the step of identifying a rank of the characteristic quality value of each molecule, and identifying, as the candidate molecule, a molecule that is at or above a predetermined rank.
[G16]
The method according to any one of G1 to G15, wherein the step of searching for a candidate molecule comprises the step of selecting at least one molecule, in which the characteristic predicted value and the estimate of uncertainty of prediction meet predetermined conditions, respectively, from the building block combination information collection.
[G17]

The method according to any one of G1 to G16, wherein the building block combination information collection is acquired using a combination optimization algorithm, and
the method further comprises a procedure of updating an extraction parameter of the combination optimization algorithm on the basis of the characteristic predicted value of the molecule and the characteristic quality value.

[G18]
The method according to G17, wherein the combination optimization algorithm is a tree-structured Parzen estimator.
[G19]
The method according to any one of G1 to G18, comprising a selection step of selecting a candidate molecule having the best experimental value among experimental values of characteristics of a plurality of candidate molecules.
[H1]
An information processing method in an information processing system for identifying a molecule suitable for a drug candidate, the method comprising the steps of:

acquiring, in accordance with a combination optimization algorithm, a building block combination information collection which is a collection of building block combination information on a plurality of different molecules;
calculating, with respect to a molecule forming an element of the building block combination information collection, a characteristic predicted value of the molecule using a prediction model for predicting a characteristic of a molecule from building block combination information on the molecule;
searching for a molecule having a more desired characteristic on the basis of the characteristic predicted value; and
updating an extraction parameter of the combination optimization algorithm on the basis of the characteristic predicted value of the molecule so as to include a molecule having a more desired characteristic.

[H2]
The information processing method according to H1, comprising the step of searching for a candidate of a molecule having a desired characteristic on the basis of a first characteristic predicted value calculated with respect to a molecule forming an element of a first building block combination information collection which is a building block combination information collection acquired before the extraction parameter is updated, and a second characteristic predicted value calculated with respect to a molecule forming an element of a second building block combination information collection which is a building block combination information collection acquired after the extraction parameter is updated.
[H3]
The information processing method according to H2, wherein the first building block combination information collection and the second building block combination information collection are collections including building block combination information on different molecules.
[H4]
The information processing method according to any one of H1 to H3, wherein the building block combination information is information on sequences of the molecules.
[H5]
The information processing method according to any one of H1 to H4, wherein the molecule is at least one of a nucleic acid, a peptide, a cyclic peptide, a protein, an antibody, and a low-molecular compound.
[H6]
The information processing method according to any one of H1 to H5, wherein the molecule is a protein, an antibody, a peptide or a cyclic peptide, and the building block combination information is information on amino acid sequences.

[H7]
The information processing method according to H6, wherein the characteristic is at least one of binding ability, pharmacological activity, a physical property, kinetics, and safety.
[H8]
The information processing method according to any one of H1 to H7, wherein the molecule is a molecule that binds to a target molecule, and the characteristic is ability to bind to the target molecule.
[H9]
The information processing method according to any one of H1 to H8, comprising the step of calculating the characteristic predicted value, and an estimate of uncertainty of the prediction.
[H10]

The information processing method according to H9, comprising the step of calculating a characteristic quality value on the basis of the characteristic predicted value and the estimate of uncertainty of prediction,
wherein the step of searching comprises the step of identifying at least one molecule from the building block combination information collection on the basis of the characteristic quality value.

[H11]
The information processing method according to H10, wherein the characteristic quality value increases with an increase in the characteristic predicted value, and decreases with a decrease in the estimate of uncertainty of prediction.
[H12]
The information processing method according to H11, wherein the characteristic quality value is an output value of a predetermined function in which two variables, one of which is the characteristic predicted value and the other of which is the estimate of uncertainty of prediction, are inputs.
[H13]
The information processing method according to any one of H10 to H12, wherein the step of calculating a characteristic quality value comprises the step of calculating a mean variance as an objective function that gives the characteristic quality value.
[H14]
The information processing method according to any one of H9 to H13, wherein the estimate of uncertainty of prediction is a standard deviation of the characteristic predicted value.
[H15]
The information processing method according to any one of H1 to H14, wherein a tree-structured Parzen estimator is used as the combination optimization algorithm pertaining to updating of the extraction parameter.
[I1]
A program configured to cause a computer to execute:

an acquisition procedure of acquiring, in accordance with a combination optimization algorithm, a building block combination information collection which is a collection of building block combination information on a plurality of different molecules;
a prediction procedure of calculating, using a prediction model for predicting a characteristic of a molecule from building block combination information on the molecule, a characteristic predicted value of a molecule forming an element of the building block combination information collection;
an updating procedure of updating an extraction parameter of the combination optimization algorithm on the basis of the characteristic predicted value of each molecule so as to include a molecule having a more desired characteristic; and
a searching procedure of searching for a molecule having a desired characteristic on the basis of the characteristic predicted value.

[I2]
The program according to I1, wherein the acquiring procedure comprises:

a procedure of acquiring a first building block combination information collection which is a collection of building block combination information on a plurality of different molecules before the extraction parameter is updated; and
a procedure of further acquiring a second building block combination information collection which is a collection of building block combination information on a plurality of different molecules after the extraction parameter is updated,

the prediction procedure comprises:

a procedure of calculating, with respect to a molecule forming an element of the first building block combination information collection, a first characteristic predicted value using the prediction model; and
a procedure of calculating, with respect to a molecule forming an element of the second building block combination information collection, a second characteristic predicted value using the prediction model,
the searching procedure comprises
a searching procedure of searching for a candidate of a molecule having a desired characteristic from the first building block combination information collection and the second building block combination information collection on the basis of the first characteristic predicted value and the second characteristic predicted value.

[I3]
The program according to I2, wherein the first building block combination information collection and the second building block combination information collection are collections including building block combination information on different molecules.
[I4]
The program according to any one of I1 to 13, wherein the building block combination information is information on sequences of the molecules.
[I5]
The program according to any one of I1 to I4, wherein the molecule is at least one of a nucleic acid, a peptide, a cyclic peptide, a protein, an antibody, and a low-molecular compound.
[I6]
The program according to any one of I1 to I5, wherein the molecule is a protein, an antibody, a peptide or a cyclic peptide, and the building block combination information is information on amino acid sequences.
[I7]
The program according to any one of I1 to I6, wherein the characteristic is at least one of binding ability, pharmacological activity, a physical property, kinetics, and safety.
[I8]
The program according to any one of I1 to I7, wherein the molecule is a molecule that binds to a target molecule, and the characteristic is ability to bind to the target molecule.
[I9]
The program according to any one of I1 to I8, comprising a procedure of calculating the characteristic predicted value, and calculating an estimate of uncertainty of the prediction.
[I10]

The program according to I9, further comprising a procedure of calculating a characteristic quality value on the basis of the characteristic predicted value and the estimate of uncertainty of prediction,
wherein the searching procedure comprises
a procedure of identifying at least one molecule from the building block combination information collection on the basis of the characteristic quality value.

[I11]
The program according to I10, wherein the characteristic quality value increases with an increase in the characteristic predicted value, and decreases with a decrease in the estimate of uncertainty of prediction.
[I12]
The program according to I11, wherein the characteristic quality value is an output value of a predetermined function in which two variables, one of which is the characteristic predicted value and the other of which is the estimate of uncertainty of prediction, are inputs.
[I13]
The program according to any one of I10 to I12, wherein the procedure of calculating a characteristic quality value comprises calculating a mean variance as an objective function that gives the characteristic quality value.
[I14]
The program according to any one of I9 to I13, wherein the estimate of uncertainty of prediction is a standard deviation of the characteristic predicted value.
[I15]
The program according to any one of I1 to I14, wherein a tree-structured Parzen estimator is used as the combination optimization algorithm pertaining to updating of the building block combination information collection.
[I16]

The program according to any one of I1 to I15, wherein the prediction model is a model learned on the basis of training data representing building block combination information different depending on molecules, and characteristics of the molecules.

[J1]

A method for producing a molecular compound, the method comprising:

an acquisition step of acquiring, in accordance with a combination optimization algorithm, a building block combination information collection which is a collection of building block combination information on a plurality of different molecules;
an input step of inputting the building block combination information collection to a prediction model;
an updating step of updating, on the basis of a characteristic predicted value of each molecule which is contained in the building block combination information collection and output from the prediction model, an extraction parameter of the combination optimization algorithm so as to include a molecule having a more desired characteristic;
a step of searching for a molecule having a more desired characteristic from the building block combination information collection, on the basis of the characteristic predicted value, followed by identification as a candidate molecule;
an output step of outputting building block combination information on the candidate molecule; and
a generation step of generating the molecular compound having a molecular sequence represented by the building block combination information.

[J2]

The method according to J1, comprising the step of searching for the candidate molecule on the basis of a first characteristic predicted value calculated with respect to a molecule forming an element of a first building block combination information collection which is a building block combination information collection acquired before the extraction parameter is updated, and a second characteristic predicted value calculated with respect to a molecule forming an element of a second building block combination information collection which is a building block combination information collection acquired after the extraction parameter is updated.

[J3]

The method according to J2, wherein the first building block combination information collection and the second building block combination information collection are collections including building block combination information on different molecules.

[J4]

The method according to any one of J1 to J3, wherein the candidate molecule has a biological sequence.

[J5]

The method according to any one of J1 to J4, wherein the building block combination information is sequence information on the molecule.

[J6]

The method according to any one of J1 to J5, wherein the molecule is at least one of a nucleic acid, a peptide, a cyclic peptide, a protein, an antibody, and a low-molecular compound.

[J7]

The method according to any one of J1 to J6, wherein the molecule is a protein, an antibody, a peptide or a cyclic peptide, and the building block combination information is information on amino acid sequences.

[J8]

The method according to any one of J1 to J7, wherein the characteristic is at least one of binding ability, pharmacological activity, a physical property, kinetics, and safety.

[J9]

The method according to any one of J1 to J8, wherein the molecule is a molecule that binds to a target molecule, and the characteristic is ability to bind to the target molecule.

[J10]

The method according to any one of J1 to J9, comprising the step of calculating the characteristic predicted value, and an estimate of uncertainty of the prediction.

[J11]

The method according to J10, comprising the step of calculating a characteristic quality value on the basis of the characteristic predicted value and the estimate of uncertainty of prediction,
wherein the inference step of searching for a candidate molecule comprises the step of identifying, as the candidate molecule, at least one molecule from the building block combination information collection on the basis of the characteristic quality value.

[J12]
The method according to J11, wherein the characteristic quality value increases with an increase in the characteristic predicted value, and decreases with a decrease in the estimate of uncertainty of prediction.
[J13]
The method according to J12, wherein the characteristic quality value is an output value of a predetermined function in which two variables, one of which is the characteristic predicted value and the other of which is the estimate of uncertainty of prediction, are inputs.
[J14]
The method according to any one of J11 to J13, wherein the step of calculating a characteristic quality value comprises calculating a mean variance as an objective function that gives the characteristic quality value.
[J15]
The method according to any one of J10 to J14, wherein the estimate of uncertainty of prediction is a standard deviation of the characteristic predicted value.
[J16]
The method according to any one of J1 to J15, wherein a tree-structured Parzen estimator is used as the combination optimization algorithm pertaining to updating of the building block combination information collection.
[J17]
The method according to any one of J1 to J16, comprising the step of selecting a candidate molecule having the best experimental value among experimental values of characteristics of a plurality of candidate molecules.
[K]

A computer system comprising a processor and a memory, wherein the memory is configured to store one or more commands, and
the commands cause the processor to:

calculate, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
search for a candidate of a molecule having a more desired characteristic on the basis of the characteristic predicted value and the uncertainty of prediction.

[L]
A non-transitory computer-readable storage medium for storing one or more commands, wherein the commands cause a computer to:

calculate, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
search for a candidate of a molecule having a more desired characteristic as a candidate molecule on the basis of the characteristic predicted value and the uncertainty of prediction.

[M]

A computer system comprising a processor and a memory, wherein the memory is configured to store one or more commands, and
the commands cause the processor to:

acquire, in accordance with a combination optimization algorithm, a building block combination information collection which is a collection of building block combination information on a plurality of different molecules;
calculate, using a prediction model for predicting a characteristic of a molecule from building block combination information on the molecule, a characteristic predicted value of a molecule forming an element of the building block combination information collection;
update an extraction parameter of the combination optimization algorithm on the basis of the characteristic predicted value of each molecule so as to include a molecule having a more desired characteristic; and search for a molecule having a desired characteristic on the basis of the characteristic predicted value.

[N]
A non-transitory computer-readable storage medium for storing one or more commands, wherein the commands cause a computer to:

acquire, in accordance with a combination optimization algorithm, a building block combination information collection which is a collection of building block combination information on a plurality of different molecules;
calculate, using a prediction model for predicting a characteristic of a molecule from building block combination information on the molecule, a characteristic predicted value of a molecule forming an element of the building block combination information collection;
update an extraction parameter of the combination optimization algorithm on the basis of the characteristic predicted value of each molecule so as to include a molecule having a more desired characteristic; and
search for a molecule having a desired characteristic on the basis of the characteristic predicted value.

[Advantageous Effect of Invention]

According to an aspect of the present disclosure, the burden accompanying drug discovery can be further reduced.

[Brief Description of Drawings]

[Figure 1] Figure 1 shows a diagram illustrating an example of a drug discovery system comprising a molecular design device according to a first embodiment.
[Figure 2] Figure 2 shows a diagram illustrating an example of a drug discovery system comprising a molecular design device according to a second embodiment.
[Figure 3] Figure 3 shows an illustration for describing an example of sequence information on a plurality of molecules in an embodiment of the present application.
[Figure 4] Figure 4 shows a diagram illustrating an example of a hardware configuration of a molecular design device in an embodiment of the present application.
[Figure 5] Figure 5 shows a diagram illustrating an example of a configuration of a controller in an embodiment of the present application.
[Figure 6] Figure 6 shows a flowchart illustrating an example of a flow of processing executed by the molecular design device in the first embodiment.
[Figure 7] Figure 7 shows a flowchart illustrating an example of a flow of processing executed by the molecular design device in the second embodiment.
[Figure 8] Figure 8 shows a flowchart illustrating an example of combination optimization processing in the second embodiment.
[Figure 9] Figure 9 shows a flowchart illustrating another example of combination optimization processing in the second embodiment.
[Figure 10] Figure 10 shows a schematic diagram of a configuration of an optimization process according to an example of application in an embodiment of the present application.
[Figure 11] Figure 11 shows a diagram illustrating an example of calculation of a target function value by optimization with TPE according to a first verification example.
[Figure 12] Figure 12 shows a diagram illustrating a relationship between a predicted mean value and a predicted standard deviation value of sequences obtained by sampling with TPE according to the first verification example.
[Figure 13] Figure 13 shows a diagram illustrating a density distribution of edit distances of sequences obtained by sampling with TPE according to the first verification example.
[Figure 14] Figure 14 shows a diagram illustrating a distribution of pseudo-correct solution model scores with respect to proposed sequences according to the first verification example.
[Figure 15] Figure 15 shows a diagram illustrating an edit distance of proposed sequences according to the first verification example.
[Figure 16] Figure 16 shows a diagram illustrating a predicted standard deviation value of proposed sequences according to the first verification example.
[Figure 17] Figure 17 shows a table showing an example of candidates of amino acids at respective mutation candidate positions in a search space according to the first verification example.

[Figure 18] Figure 18 shows a table showing an example of a parameter configuration of TPE according to the first verification example.

[Figure 19] Figure 19 shows a diagram illustrating distributions of predicted mean values and predicted standard deviation values of sequences obtained by sampling with TPE according to a second verification example.

[Figure 20] Figure 20 shows a t-SNE visualization diagram illustrating sequences from sampling with TPE according to the second verification example.

[Figure 21] Figure 21 shows a diagram illustrating a predicted mean value of proposed sequences according to the second verification example.

[Figure 22] Figure 22 shows a diagram illustrating a predicted variance value of proposed sequences according to the second verification example.

[Figure 23] Figure 23 shows a diagram illustrating a distribution of expression levels of proposed sequences according to the second verification example.

[Figure 24] Figure 24 shows a diagram illustrating a distribution of an octet value of respective sequences according to the second verification example.

[Description of Embodiments]

I. Definition

In the present specification, the term "and/or" is used to show the matters described before and after "and/or", or any combination thereof. For example, "A, B and/or C" includes the matters "A", "B" and "C", and combinations "A and B", "A and C", "B and C", and "A and B and C".

- Amino Acids

In the present specification, natural amino acids and non-natural amino acids may be included. In the case of a natural amino acid, the amino acid is indicated by one-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

- Modification of Amino Acids

A known method such as site-specific mutagenesis (Kunkel et al. (Proc. Natl. Acad. Sci. USA (1985) 82, 488-492)) and Overlap extension PCR can be appropriately employed for modification of an amino acid in the amino acid sequence of an antigen-binding molecule. A plurality of methods known in the art can also be adopted as alteration methods for substituting an amino acid with an amino acid other than a natural amino acid (Annu. Rev. Biophys. Biomol. Struct. (2006) 35, 225-249; and Proc. Natl. Acad. Sci. U.S.A. (2003) 100 (11), 6353-6357). For example, a cell-free translation system (Clover Direct (ProteinExpress Co., Ltd.)) including tRNA in which a non-natural amino acid is bonded to amber suppressor tRNA complementary to a UAG codon (amber codon), one of stop codons, may also be used.

- Antigen

Herein, the structure of an "antigen" is not limited to a particular structure as long as the structure contains an epitope to which an antigen-binding domain binds. In certain modes, the antigen is a peptide of four or more amino acids, or a polypeptide, or a protein.

Examples of the antigen include membrane-associated molecules expressed on cell membranes, and soluble molecules extracellularly secreted by cells.

- Antigen-Binding Domain

Herein, domains of any structure that bind to an antigen of interest can be used as an "antibody-binding domain". Examples of such domains include variable regions of a heavy chain and a light chain of an antibody; a module called an A domain, which is contained in Avimer, a cell membrane protein present in the living body, and consists of about 35 amino acids (International Publication Nos. WO 2004/044011, WO 2005/040229); Adnectin, which contains a 10Fn3 domain, a domain that binds to the protein in fibronectin, which is a glycoprotein expressed on cell membranes (International Publication No. WO 2002/032925); Affibody, which uses, as a scaffold, an IgG-binding domain constituting three helical bundles each consisting of 58 amino acids of Protein A (International Publication No. WO 1995/001937); DARPins (Designed Ankyrin Repeat proteins), which is a region in which ankyrin repeats (ARs), each containing 33 amino acid residues, that have a structure including repeatedly stacked subunits of a turn, two antiparallel helices, and a loop are exposed to the molecular surface (International Publication No. WO 2002/020565); Anticalin, which is four loop regions supporting one side of a barrel structure in which eight highly conserved antiparallel strands twist to the central direction in a lipocalin molecule such as neutrophil gelatinase-associated lipocalin (NGAL) (International Publication No. WO2003/029462); and a concave region of a parallel sheet structure in the inside of the horseshoe-shaped structure including repeatedly stacked leucine-rich-repeat (LRR) modules of a variable lymphocyte receptor (VLR) not having the structure of immunoglobulin as an acquired immune system of agnathans such as lampreys and hagfish (International Publication No. WO 2008/016854).

Examples of the antigen-binding domain in the present disclosure include antigen-binding domains containing variable regions of a heavy chain and light chain of an antibody. Examples of such an antigen-binding domain include "scFv (single chain Fv)", "single chain antibody", "Fv", "scFv2 (single chain Fv2)", "Fab", or "F(ab')2".

- Antigen-Binding Molecule

In the present disclosure, the term "antigen-binding molecule", which contains an antigen-binding domain, is used in the broadest sense, and specifically includes various molecular types containing an antigen-binding domain. The antigen-binding molecule may be a molecule consisting only of an antigen-binding domain, or a molecule containing an antigen-binding domain and another domain. If the antigen-binding molecule is a molecule formed of an antigen-binding domain and an Fc region bound together, for example, examples of such molecules include complete antibodies and antibody fragments. Antibodies can include single monoclonal antibodies (including agonist and antagonist antibodies), human antibodies, humanized antibodies, and chimeric antibodies. The term "antigen-binding molecule" in the present disclosure includes scaffold molecules formed as a library for construction of antigen-binding domains by using only a partial structure of three-dimensional structure, as a scaffold, such as existing stable $\alpha\beta$/ barrel protein structure.

In the present specification, the term "antibody" is used in the broadest sense and encompasses various antibody structures including, but not limited to, a monoclonal antibody, a polyclonal antibody, a multispecific antibody (e.g., a bispecific antibody), and an antibody fragment as long as the antibody exhibits the desired antigen-binding activity.

The antibody can be isolated from a natural resource such as plasma and serum in which the antibody is naturally present, or a culture supernatant of hybridoma cells producing the antibody, or can be partially or completely synthesized by using a technique of gene recombination or the like. Examples of the antibody include isotypes of immunoglobulin and subclasses of these isotypes. Known as human immunoglobulin are nine classes (isotypes): IgG1, IgG2, IgG3, IgG4, IgA1, IgA2, IgD, IgE, and IgM. Among these isotypes, the term "antibody" in the present disclosure includes IgG1, IgG2, IgG3, and IgG4. While a plurality of allotype sequences resulting from genetic polymorphism are described as human IgG1, human IgG2, human IgG3, and human IgG4 constant regions in Sequences of proteins of immunological interest, NIH Publication No. 91-3242, any of them is acceptable in the present disclosure. In particular, in the case of the sequence of human IgG1, the amino acid sequence ranging from position 356 to position 358 as specified by EU numbering may be DEL or EEM. While a plurality of allotype sequences resulting from genetic polymorphism are described as human Ig$\kappa$ (Kappa) constant regions and human Ig$\lambda$ (Lambda) constant regions in Sequences of proteins of immunological interest, NIH Publication No. 91-3242, any of them is acceptable in the present disclosure.

The "antibody portion" refers to a molecule, other than a complete antibody, which contains a part of the complete antibody and binds to an antigen to which the complete antibody binds. Examples of the antibody fragment include, but are not limited to, Fv, Fab, Fab', Fab'-SH, F(ab')$_2$, diabody, linear antibodies, single-chain antibody molecules (e.g., scFv), and multispecific antibodies formed from antibody fragments.

The terms "full-length antibody", "complete antibody", and "whole antibody" are used interchangeably with each other in the present specification and refer to an antibody having a structure substantially similar to a natural antibody structure, or having heavy chains containing a Fc region defined in the present specification.

The term "variable region" or "variable domain" refers to a domain of the heavy chain or light chain of an antibody, which is involved in binding of the antibody to the antigen. The variable domains (VH and VL) of the heavy chain and the light chain of a natural antibody normally have similar structure including a framework region (FR) including four conserved domains and three hypervariable regions (HVR) (e.g., see Kindt et al. Kuby Immunology, 6th ed., W.H. Freeman and Co., page 91 (2007)). One VH or VL domain may suffice for conferring antigen binding specificity. In addition, an antibody that binds to a certain antigen may be isolated with use of the VH or VL domain from an antibody that binds to the antigen through screening of a complementary library to the VL or VH domain. For example, see Portolano et al., J. Immunol. 150:880-887 (1993); Clarkson et al., Nature 352:624-628 (1991).

As used herein, the term "molecular weight" means the sum of the atomic weights of the atoms constituting the compound molecule (unit: "g/mol"), and is obtained by calculating the sum of the atomic weights of the atoms included in the molecular structure formula. The unit of molecular weight is sometimes omitted herein. The molecular weight can be measured, for example, by liquid chromatography-mass spectrometry (LC/MS).

As used herein, the medium-molecular compound, or the medium molecule is a compound having a molecular weight of 500 g/mol or more and less than 30,000 g/mol.

The medium-molecular compound is, for example, a compound having a molecular weight of 500 g/mol or more and less than 6,000 g/mol, and may be a compound having a molecular weight of 500 g/mol or more and less than 4,000 g/mol, may be a compound having a molecular weight of 600 g/mol or more and less than 4,000 g/mol, or may be a compound having a molecular weight of 700 g/mol or more and less than 3,000 g/mol. The medium-molecular compound is a peptide compound containing a peptide chain, a nucleic acid, or a sugar chain, and may be a peptide compound, may be a peptide compound containing 5 to 30 amino acid residues, may be a peptide compound having 7 to 25 amino acid residues, or may be a peptide compound containing 9 to 20 amino acid residues. The medium-molecular compound is, for example, a peptide compound having a molecular weight of 500 g/mol or more and less than 6,000 g/mol, and may be a peptide compound having a molecular weight of 500 g/mol or more and less than 4,000 g/mol, may be a peptide compound having a molecular weight of 600 g/mol or more and less than 4,000 g/mol, or may be a peptide compound having a molecular weight of 700 g/mol or more and less than 3,000 g/mol.

As used herein, the low-molecular compound is a compound having a molecular weight of less than 500 g/mol. As used herein, the high-molecular compound is a compound having a molecular weight of 30,000 g/mol or more.

(Definition of Experimental Techniques)

[Characterization]

As a mode of the present disclosure, a prediction model is generated by performing machine learning on the basis of sequence information on molecules and evaluation result information on characterization of the molecules. Examples of a non-limiting mode of characterization of molecules include evaluation of binding ability, evaluation of pharmacological activity, evaluation of physical properties, evaluation of kinetics, and evaluation of safety of molecules, but are not limited to these evaluations.

- Evaluation of Binding Ability

The technique of evaluation of ability of a target molecule-binding molecule to bind to a target molecule is not limited in any way, and evaluation can be made by quantitatively evaluating the binding of the target molecule-binding molecule to the target molecule. The target molecule is, for example, a target protein. The target molecule-binding molecule is, for example, an antigen-binding molecule, and the target molecule is, for example, an antigen. For example, when the target molecule is an antigen, evaluation can be made by measuring binding activity of an antigen-binding molecule and an antigen. "Binding activity" refers to the total intensity of non-covalent interactions between one or more binding sites of a molecule (e.g., an antibody) and a binding partner for the molecule (e.g., an antigen). Here, "binding activity" is not strictly limited to 1:1 interaction between members of a certain binding pair (e.g., an antibody and an antigen). When members of a binding pair reflect monovalent 1:1 interaction, for example, the meaning of binding activity is intrinsic binding affinity (sometimes referred to simply as "affinity"). If members of a binding pair are capable of both monovalent binding and multivalent binding, the binding activity is the sum of avidities of them. The binding activity of a molecule, X, to its partner, Y, can be generally represented by a dissociation constant (KD) or the "amount of analyte bound per unit amount of ligand". Binding activity can be measured with any of common methods known in the art including those described herein. Those skilled in the art can appropriately determine conditions except the concentration of a compound specific to target tissue. In a particular mode, the antigen-binding molecule provided herein is an antibody, and the binding activity of the antibody is a dissociation constant (KD) of $\leq 1\ \mu M$, $\leq 100 nM$, $\leq 10\ nM$, $\leq 1 nM$, $\leq 0.1 nM$, $\leq 0.01\ nM$, or $\leq 0.001\ nM$ (e.g., $10^{-8}$ M or lower, such as $10^{-8}$ M to $10^{-13}$ M, such as $10^{-9}$ M to $10^{-13}$ M).

In a mode, for the binding activity of an antibody, a ligand capture method, for example, with a BIACORE (R) T200 or BIACORE (R) 4000 (GE Healthcare, Uppsala, Sweden), which uses surface plasmon resonance analysis as the measurement principle, is used. For the machine operations, for example, BIACORE (R) Control Software is used. In a mode, an amine coupling kit (GE Healthcare, Uppsala, Sweden) is used in accordance with instruction from the supplier to immobilize molecules for ligand capture such as an anti-tag antibody, an anti-IgG antibody, and protein A on a sensor chip coated with carboxymethyldextran (GE Healthcare, Uppsala, Sweden). The ligand capture molecules are diluted with 10 mM sodium acetate solution at an appropriate pH, and injected at an appropriate flow rate in an appropriate injection time. In measurement of binding activity, a buffer containing 0.05% polysorbate 20 (another name: Tween (R)-20) is used as a buffer for measurement, and measurement is performed at a flow rate of 10 to 30 μL/min at a measurement temperature of, for example, 25°C or 37°C. When measurement is carried out by allowing the molecules for ligand capture to capture an antibody as a ligand, the antibody is injected to allow an intended amount of the antibody to be captured, and a serially diluted product (analyte) of an antigen and/or Fc receptor prepared with the buffer for measurement is then injected. When measurement is carried out by allowing the molecules for ligand capture to capture an antigen and/or Fc receptor as a ligand, the antigen and/or Fc receptor are/is injected to allow an intended amount of the antigen and/or Fc receptor to be captured, and a serially diluted product (analyte) of an antibody prepared with the buffer for measurement is then injected.

In a mode, measurement results are analyzed by using BIACORE (R) Evaluation Software. In carrying out calculation of kinetics parameters, sensorgrams for association and dissociation are simultaneously fit with use of a 1:1 Binding model, and association rates (kon or ka), dissociation rates (koff or kd), and equilibrium dissociation constants (KD) can be calculated. If the binding activity is weak, in particular, if calculation of kinetics parameters is difficult because of fast dissociation, the equilibrium dissociation constant (KD) may be calculated with use of a Steady state model. As another parameter for binding activity, the "amount of analyte bound per unit amount of ligand" can also be calculated by dividing the amount of a bound analyte at a specific concentration (RU) by the amount of the captured ligand (RU).

If the antigen is a soluble molecule, KD (dissociation rate constant) can be used as a value for antigen-binding activity; if the antigen is a membrane-associated molecule, apparent kd (apparent dissociation rate constant) can be used. kd (dissociation rate constant) and apparent KD (apparent dissociation rate constant) can be measured with a method known to those skilled in the art, and, for example, Biacore (GE healthcare), a flow cytometer, and so on can be used.

Another mode of characterization is, for example, a selection technique for antigen-binding molecules with use of a display library. In a mode, an example is panning using phage display. In evaluation of affinity, for example, a phage presenting an antigen-binding molecule that interacts with a target antigen can be concentrated through a process in which a phage library presenting different antigen-binding molecules is prepared, a target antigen is brought into contact with the phages prepared, and an operation to wash out unbound phages is then carried out. A sequence having affinity with the target antigen can be identified by analyzing the nucleic acid sequence encoding the antigen-binding molecule contained in the concentrated phage. In another mode, an example is panning using mammalian cell display. In evaluation of pharmacological activity using the display system, for example, cells having an antigen-binding molecule gene with desired pharmacological activity can be isolated by using a flow cytometer or the like through a process in which a library containing a plurality of different antigen-binding molecules is expressed in targeted mammalian cells and reporter activity and so on are changed according to the action exhibited thereby on the cells. In evaluation of physical properties using the display system, for example, cells having an antigen-binding molecule gene capable of stably expressing at high level can be isolated by using a flow cytometer or the like through a process in which a library containing a plurality of different antigen-binding molecules is expressed in targeted mammalian cells and the expression levels are examined by staining with antibodies specific to the antigen-binding molecules. Characterization of antigen-binding molecules by panning is not limited to the techniques using phages or mammalian cells, and various techniques can be used as long as they allow presentation of antigen-binding molecules, and examples thereof include, but are not limited to, a technique to allow ribosomes to present, a technique to allow mRNA to present, a technique to allow viruses other than phages to present, and a technique to allow bacteria such as Escherichia coli to present.

Another mode of characterization is, for example, a method of obtaining an antibody gene sequence from immunocytes derived from an individual or a method of obtaining an antibody protein sequence from serum. In evaluation of affinity involving extraction of an antibody gene sequence from immunocytes, for example, a sequence having affinity with a target antigen can be identified through a process involving administering a target antigen protein to an individual to induce immune sensitization, and extracting an antibody gene for an antibody that binds to the target antigen from immunocytes having the gene.

The antigen to cause immune sensitization is not limited to a protein as in the technique and a gene encoding the protein or cells expressing the protein can be used. Examples of the individual as a subject include humans, mice, rats, hamsters, rabbits, monkeys, chickens, camels, llamas, and alpacas, but are not limited thereto.

Examples of techniques for the analysis of nucleic acid sequences or appearance frequency include a technique in which gene recombinant organisms having nucleic acid sequences for different antigen-binding molecules are cloned and analysis is performed through the Sanger method using capillary electrophoresis, and a technique to analyze with use of a next-generation sequencer, but are not limited thereto.

In the analysis of nucleic acid sequences, strength of a characteristic can be additionally determined on the basis of appearance frequency. For example, the characteristic of an antigen-binding molecule encoded by a sequence with high appearance frequency in analysis of nucleic acid sequences after concentration can be estimated to be high, and the characteristic of an antigen-binding molecule encoded by a sequence with low appearance frequency after concentration can be estimated to be lower than that of an antigen-binding molecule encoded by a sequence with high appearance frequency.

The techniques to acquire information on antigen-binding molecules derived from the display library or an individual are applicable to various types of characterization, and not limited to the above.

- Evaluation of Pharmacological Activity

The technique of evaluation of pharmacological activity for molecules is not limited in any way, and evaluation can be made, for example, by measuring neutralization activity, agonist activity, or cytotoxic activity exhibited by molecules. In evaluation of cytotoxic activity as an example of pharmacological activity, examples of cytotoxic activity to be evaluated include antibody-dependent cell-mediated cytotoxicity (ADCC) activity, complement-dependent cytotoxicity (CDC) activity, T-cell-dependent cytotoxicity (TDCC) activity, and antibody-dependent cellular phagocytosis (ADCP) activity. CDC activity is cytotoxic activity due to the complement system. ADCC activity is such activity that an immunocyte or the like binds to the Fc region of an antigen-binding molecule containing an antigen-binding domain that binds to a membrane-associated molecule expressed on the cell membrane of a target cell via the Fcγ receptor expressed on the immunocyte, and the immunocyte causes damage to the target cell. TDCC activity is such activity that a T cell causes disorder to a target cell through bringing the target cell and the T cell into close proximity with use of a bi-specific antibody containing an antigen-binding domain that binds to a membrane-associated molecule expressed on the cell membrane of the target cell and an antigen-binding domain against any of the constituent subunits of the T cell receptor (TCR) complex on the T cell, in particular, an antigen-binding domain that binds to the CD3 epsilon chain. Whether an antigen-binding molecule of interest has ADCC activity, CDC activity, TDCC activity, or ADCP activity can be determined by a known method.

Neutralization activity is activity that is against ligands having biological activity against cells, such as viruses and toxins, and inhibits the biological activity. That is, a substance having neutralization activity is a substance that binds to such a ligand or a receptor to which the ligand binds to inhibit binding between the ligand and the receptor. A receptor that has been prevented from binding to the ligand by neutralization activity comes to be disabled from exerting the biological activity mediated by the receptor. When the antigen-binding molecule is an antibody, an antibody having such neutralization activity is generally called a neutralizing antibody, and the neutralization activity can be determined by measuring the inhibitory activity to binding between the ligand and a receptor. The ligand having biological activity to cells is not limited to viruses, toxins, and so on, and inhibitory activity to physiological action evoked by binding of an endogenous ligand such as cytokine and chemokine to a receptor is also understood as neutralization activity. Neutralization activity is not limited to cases with inhibition of binding between a ligand and a receptor, and activity to inhibit the function of a protein having biological activity is also understood as neutralization activity, and examples of the function of a protein include enzymatic activity.

- Evaluation of Physical Properties

The technique of evaluation of physical properties for molecules is not limited in any way, and examples of physical properties include thermal stability, chemical stability, solubility, viscosity, photostability, long-term storage stability, non-specific adsorptivity, lipophilicity, and membrane permeability. In evaluation of the various physical properties exemplified, they can be measured with methods known to those skilled in the art. The evaluation methods are not limited in any way, and, in evaluation of stability such as thermal stability, chemical stability, photostability, stability to mechanical stimulation, and long-term storage stability, for example, evaluation can be made by measuring the decomposition, chemical modification, and association of a molecule of interest before and after treatment intended for the evaluation of stability such as heat treatment, exposure to a low-pH environment, exposure to light, stirring with a machine, and long-term storage. Examples of one non-limiting mode of the measurement method involving such evaluation of stability include techniques using chromatography such as ionexchange chromatography and size exclusion chromatography, mass spectrometry, and electrophoresis, but are not limited thereto, and measurement can be performed with various techniques known to those skilled in the art.

Examples of evaluation of physical properties other than the above-described evaluations include evaluation of solubility of protein by a polyethylene glycol precipitation method, evaluation of viscosity by a small-angle X-ray scattering method, and evaluation of non-specific binding based on evaluation of binding to the Extracellular Matrix (ECM), but are not limited thereto.

Even for evaluation of protein expression levels, evaluation of binding to a resin for purification or ligand for purification, and evaluation of surface electric charge, evaluation can be made as evaluation of physical properties as long as measurement can be performed with a technique known to those skilled in the art.

- Evaluation of Kinetics

The technique of evaluation of kinetics for molecules is not limited in any way, and evaluation can be made by administering a molecule to an animal such as a mouse, a rat, a monkey, and a dog and measuring the amount of the molecule in the blood after administration over time, and evaluation can be made with a technique widely known to those skilled in the art as pharmacokinetics (PK) evaluation. In addition to the technique to directly evaluate PK, the behavior of a molecule in kinetics can be predicted from the amino acid sequence of a molecule by calculating the surface electric charge, isoelectric point, and so on of the molecule with software.

- Evaluation of Safety

The technique of evaluation of safety for molecules is not limited in any way, and examples thereof include an immunogenicity prediction tool such as ISPRI Web-Based Immunogenicity Screening (EpiVax, Inc.), evaluation of HLA binding of fragment peptides of an antigen-binding molecule, detection of a T-cell epitope by using MAPPs (MHC-Associated Peptide Proteomics) or evaluation of T-cell growth, and evaluation of immunogenicity. Evaluation can be made as long as measurement can be performed with a technique known to those skilled in the art such as evaluation of immunoreaction using binding to the rheumatoid factor (RF), PBMC, or whole blood and evaluation of platelet aggregation.

(Definitions of Terms and Techniques Used in Machine Learning)

- MBO (Model Based Optimization)

MBO (model based optimization) means that the characteristic value of a characteristic is not itself a direct target to be optimized, but a predicted value of the characteristic value of a characteristic which is estimated by some model is a target to be optimized.

- TPE (Tree-Structured Parzen Estimator)

TPE (tree-structured Parzen estimator) is a type of Bayesian optimization. TPE includes a process in which for a function to be optimized, an expected improvement in an output value given a certain input value is calculated on the basis of a conditional probability of an output value given an input value, and a probability to an output value. That is, TPE is a technique of optimization of a function using an input value which maximizes an expected improvement calculated as described above.

(Method for Producing Target)

In an aspect of the present disclosure, a method for producing a target suitable for a drug candidate, which is acquired, can be carried out by a method well known to those skilled in the art.

When the target is an antibody, the antibody can be produced by, for example, a method or a configuration of recombination as described in U.S. Patent No. 4,816,567. A mode is a method for preparing an antibody, comprising culturing, under conditions suitable for expression of an antibody which is a candidate molecular compound described herein, host cells containing a nucleic acid encoding the antibody, and optionally collecting the antibody from the host cells (or a host cell culture medium). The isolated nucleic acid encoding the antibody may encode an amino acid sequence containing VL of the antibody and/or an amino acid sequence containing VH of the antibody (for example, a light chain and/or a heavy chain of the antibody). Host cells containing such a nucleic acid contain (1) a vector containing a nucleic acid encoding an amino acid sequence containing VL of the antibody and an amino acid sequence containing VH of the antibody, or (2) a first vector containing a nucleic acid encoding an amino acid sequence containing VL of the antibody and a second vector containing a nucleic acid encoding an amino acid sequence containing VH of the antibody (for example, the host cells are transformed). In a mode, host cells are eukaryotic (for example, Chinese hamster ovary (CHO) cells) or lymphatic cells (for example, Y0, NS0, Sp2/0 cells)). Host cells suitable for cloning or expression of a vector encoding an antibody include prokaryotic cells or eukaryotic cells. For example, the antibody may be produced with bacteria particularly when glycosylation and an Fc effector function are not required. For expression of antibody fragments and polypeptides in bacteria, see, for example, U.S. Patent Nos. 5,648,237, 5,789,199 and 5,840,523 (see also Charlton, Methods in Molecular Biology, Vol. 248 (B.K.C. Lo, ed., Humana Press, Totowa, NJ, 2003), pp. 245-254). After the expression, the antibody may be isolated from a bacterial cell paste into a soluble fraction, and can be further purified.

When the target is a peptide compound, or a cyclic peptide compound, it can be produced by liquid-phase synthesis, a solid-phase synthesis involving Fmoc synthesis, Boc synthesis or the like, or a combination thereof. The liquid-phase synthesis and solid-phase synthesis can be performed by methods well known to those skilled in the art. The solid synthesis is a method in which a compound is bound to a solid, and the compound and a reagent is chemically reacted on the solid resin to synthesize an intended compound. The solid-phase synthesis of a peptide is a method in which a desired amino acid or peptide is bound to a solid resin, and to the amino acid or the peptide bound to the solid resin, desired amino acids or peptides are sequentially linked to extend the peptide chain, thereby synthesizing a peptide. By separating, from the solid resin, the peptide bound to the solid resin, an intended peptide can be obtained.

(First Embodiment)

Figure 1 shows a block diagram illustrating an example of a drug discovery system 100 comprising a molecular design device 1 according to a first embodiment.

The drug discovery system 100 is a system for creating a new target suitable for a drug candidate. The present system provides a method for generating a new target having a predetermined characteristic such as specific physiological activity (for example, binding to a specific protein). Examples of the drug include, but are not limited to, potential activators such as low-molecular pharmaceuticals, medium-molecular pharmaceuticals, biological agents, cells, nucleic acid pharmaceuticals, biopharmaceuticals, and other activators. The target includes a molecular structure or the like having desired or defined biological activity (for example, binding to a specific protein in preference to other proteins). Molecules as drug candidates include biological molecules, and compounds, with the inclusion of various molecules such as nucleic acids, peptides, cyclic peptides, proteins, antibodies, target molecule-binding molecules, high-molecular compounds, medium-molecular compounds, and low-molecular compounds.

The drug discovery system 100 may comprise, for example, a device for selecting a molecule that interacts with a target for an agent, and a device for creating a lead molecule, which are not described herein. The drug delivery system 100 may be, for example, an information processing system comprising the disclosure of WO 2020/246617.

The drug discovery system 100 comprises the molecular design device 1. The molecular design device 1 searches for a candidate of a molecule having a desired characteristic, and outputs information on the identified candidate. The information that is output is building block combination information on the candidate molecule. In other words, the molecular design device 1 identifies a candidate molecule, and outputs building block combination information on the identified candidate molecule. Here, the candidate molecule is a molecule expected to have a desired characteristic. The candidate molecule building block combination information is information on a candidate molecule, which is building block combination information on a part or the whole of the candidate molecule. The candidate molecule building block combination information that is output may include information representing one candidate molecule, or may include information representing a plurality of candidate molecules.

The drug discovery system 100 selects a new target suitable for a drug candidate using the candidate molecule building block combination information output from the molecular design device 1. The drug discovery system 100 generates a candidate molecule on the basis of the candidate molecule building block combination information output from the molecular design device 1, experimentally evaluates a characteristic of the candidate molecule, and selects a molecule having a desired characteristic as a new target suitable for a drug candidate on the basis of the results of characteristic evaluation. That is, using the candidate molecule building block combination information output by the molecular design device 1, the drug discovery system 100 can create a new target suitable for a drug candidate on the basis of the results of evaluating the characteristic as the candidate molecule is actually generated. In this case, the candidate molecule can be said to be a molecule against which verification can be performed for narrowing down candidates for a main component of a pharmaceutical.

The building block combination information on a molecule is information on combinations of building blocks of a part or all of the molecule. When the building block combination information is information on combinations of building blocks of a part of the molecule, it may be possible to arbitrarily set a range of sequences thereof.

The building block is a unit that constitutes a molecule. The building block combination information on a molecule relates to information on combinations of building blocks constituting a molecule. In the present application, a sequence comprising individual constituent elements may be referred to as a "combination". The term "sequence" may be used as an example of the building block combination.

The molecule designed is, for example, a protein. When the molecule is a protein, the building block is an amino acid, and the building block combination information on the molecule is, for example, information on the amino acid sequence of the protein.

The molecule designed is, for example, a nucleic acid. When the molecule is a nucleic acid, the building block is a nucleotide. The sequence of the molecule is, for example, a nucleotide sequence of the nucleic acid. The building block combination information on the molecule is information about the nucleotide sequence.

More specifically, when the molecule designed is an antibody, the molecular sequence is an amino acid sequence, and the building block is an amino acid. The building block combination information on the molecule is, for example, a full-length sequence of the antibody, for example, an amino acid sequence of VH or VL, or a sequence of a part of the antibody, such as CDR or FR.

For example, the molecule designed is a cyclic peptide, the molecular sequence is an amino acid sequence including non-natural amino acids, and the building blocks are natural amino acids and non-natural amino acids. The building block combination information on the molecule is information on the amino acid sequence including non-natural amino acids.

When the molecule designed is a small molecule, the building block combination of the molecule is a combination of fragments, and the building block is a fragment (a molecule of a fragment constituting the small molecule).

For example, when the molecule designed is a nucleic acid, the molecular sequence is a nucleotide sequence, and the building block is a base.

The desired characteristic is a characteristic required for a new target suitable for a drug candidate, and can be arbitrarily set. Examples of a non-limiting mode of the characteristic include, but are not limited to, ability to bind to a predetermined *in vivo* target, binding ability, pharmacological activity, physical properties, kinetics, and safety. For example, when the molecule designed is an antibody, the characteristic is, for example, the ability of an agent to bind to a predetermined antigen. For example, when the molecule designed is mRNA (messenger-RNA), the characteristic is, for example, ability to translate a protein.

The molecular design device 1 is an example of an inference device for inferring a molecule having a desired characteristic from prediction information that will be described later. Examples of the desired characteristic include having ability to bind to a target molecule, having efficacy, and having medicative features including membrane permeability. Hereinafter, inferring a molecule having a desired characteristic from prediction information may be rephrased as identifying a candidate molecule.

In the present embodiment, the molecule expected to have a desired characteristic is a molecule which, with respect to a desired characteristic, shows a good predicted value with a prediction model, and low prediction uncertainty.

Hereinafter, the case in which sequence information is processed as building block combination information will be described.

The molecular design device 1 comprises an inferrer 111 comprising, for example, a sequence information processor 111a, a characteristic predictor 111b, a prediction information processor 111c, and a candidate molecule identifier 111d.

The sequence information processor 111a provides a sequence information collection, and outputs the provided sequence information collection to the characteristic predictor 111b. The sequence information collection is a collection of sequence information on a plurality of molecules. The sequence information processor 111a may autonomously generate sequence information on each molecule, or may input sequence information on each molecule from another equipment. The sequence information collection may be rephrased as a building block combination information collection, and the sequence information processor 111a may be rephrased as a building block combination information processor.

The characteristic predictor 111b predicts, with respect to sequence information on a molecule which is each element of the sequence information collection input from the sequence information processor 111a, a characteristic of the molecule, and outputs prediction information pertaining to the predicted characteristic to the prediction information processor 111c.

The prediction information processor 111c acquires prediction information on each molecule from the characteristic predictor 111b. The prediction information processor 111c outputs the acquired prediction information to the candidate molecule identifier 111d.

The candidate molecule identifier 111d identifies sequence information on at least one candidate molecule on the basis of the prediction information. The candidate molecule identifier 111d may output, to another equipment, output data representing the identified sequence information, or may store the output data in a storage 14 (described later).

In the present embodiment, the sequence information collection may be a collection of sequence information representing virtual molecular sequences (sometimes referred to as "virtual sequences") generated by machine learning, may be a collection of sequence information on real molecular sequences (sometimes referred to as "actual sequences"), or may be a collection including both sequence information representing virtual sequences and sequence information representing actual sequences. For example, the sequence information collection may include sequence information on virtual sequences generated by a virtual sequence generation model. The sequence information collection may include sequence information representing actual sequences obtained as, for example, existing databases and experimental results. The sequence information collection may include sequence information extracted from all candidates of combinations of building blocks by combination optimization that will be described later.

The sequence information processor 111a performs processing to provide a sequence information collection. The sequence information processor 111a may further comprise a sequence information collection acquirer (not shown) configured to execute an acquisition process for acquiring sequence information as an output with respect to certain input information using a machine learning model. In this case, specifically, the sequence information processor 111a acquires the sequence information collection, and outputs the acquired sequence information collection to the characteristic predictor 111b.

In the present embodiment, sequence information on a plurality of molecules is input from the sequence information processor 111a to the characteristic predictor 111b.

The characteristic predictor 111b computes characteristic predicted values and estimates of uncertainty of prediction for input sequence information on a plurality of molecules, respectively.

In the present embodiment, the characteristic predictor 111b comprises a predicted value calculator 111x, and a prediction uncertainty estimator 111y. The predicted value calculator 111x inputs sequence information on molecules, and calculates a characteristic predicted value using a prediction model. The characteristic predicted value is a characteristic value predicted using the prediction model. The prediction uncertainty estimator 111y estimates uncertainty of the characteristic predicted value calculated by the predicted value calculator 111x. The value representing the estimated uncertainty is referred to as an estimate of uncertainty of prediction.

The prediction model is generated by, for example, learning based on training data including a plurality of sets of sequence information on each molecule and an evaluated characteristic of the molecule.

The predicted value calculator 111x may calculate the characteristic predicted values of a plurality of characteristics. When the characteristic values of a plurality of characteristics are predicted, the prediction uncertainty estimator 111y may estimate the uncertainty of prediction for the characteristic predicted value of each characteristic, or may estimate the uncertainty of prediction for one of the characteristic predicted values.

The characteristic predictor 111b outputs a characteristic predicted value and an estimate of uncertainty of prediction for the characteristic predicted value as prediction information on each molecule to the prediction information processor 111c.

The candidate molecule identifier 111d performs processing to infer a molecule having a desired characteristic on the basis of the prediction information, and identifies a candidate molecule.

In the present embodiment, the candidate molecule identifier 111d infers a molecule having a desired characteristic on the basis of the characteristic predicted value and the estimate of uncertainty of prediction for the sequence information on each molecule which is output from the characteristic predictor 111b. The candidate molecule identifier 111d identifies, on the basis of the prediction information obtained from the prediction information processor 111c, at least one candidate molecule from a plurality of molecules whose sequence information is contained in the sequence information collection from the sequence information processor 111a. The candidate molecule identifier 111d may use the characteristic predicted value of at least one characteristic or the characteristic predicted values of a plurality of characteristics in identification of a candidate molecule. The candidate molecule identifier 111d may use a plurality of characteristic predicted values and the estimate of uncertainty of prediction for at least one characteristic predicted value as prediction information in identification of a candidate molecule. That is, in identification of a candidate molecule, a constraint on a second characteristic value (uncertainty of prediction for the characteristic predicted value) may be allowed for optimizing a first characteristic value (characteristic predicted value). The candidate molecule identifier 111d may identify, as a candidate molecule, a molecule having a desired characteristic on the basis of a characteristic quality value described later.

For example, the candidate molecule identifier 111d may select, as a candidate molecule, at least one molecule in which a characteristic predicted value and uncertainty of prediction meet predetermined conditions, respectively. The candidate molecule identifier 111d may select, as a candidate molecule, at least one molecule in which a plurality of characteristic predicted values and an estimate of uncertainty of prediction for at least one characteristic predicted value meet predetermined conditions, respectively.

The candidate molecule identifier 111d may identify at least one candidate molecule on the basis of a characteristic quality value calculated on the basis of a characteristic predicted value and an estimate of uncertainty of prediction. In this case, the characteristic quality value may be calculated in the prediction information processor 111c, or may be calculated in the characteristic predictor 111b, for example.

The characteristic predicted value is a value based on a characteristic predicted value and an estimate of uncertainty of prediction. The characteristic quality value can also be considered as an objective variable calculated using a predetermined function with a characteristic predicted value and an estimate of uncertainty of prediction as explanation variables.

For example, the characteristic quality value may be an index value which gives a smaller value with an increased estimate of uncertainty of characteristic prediction, and gives a larger value with an increased characteristic predicted value. In other words, the characteristic quality value is only required to increase with an increase in characteristic predicted value, and decrease with an increase in estimate of uncertainty of prediction. As an example, the characteristic quality value is a difference between a characteristic predicted value and a predetermined coefficient multiple of an estimate of uncertainty of prediction in a linear region, or a difference between a predetermined coefficient multiple of a characteristic predicted value and an estimate of uncertainty of prediction. The predetermined coefficient is a positive constant, and represents an extent of contribution of the characteristic value or the estimate of uncertainty of prediction to the characteristic quality value.

Therefore, when a characteristic predicted value which gives a larger value with the increased degree to which having a desired characteristic is predicted, or with an increased extent of the characteristic is defined, the characteristic quality value is an index which gives a larger value with an increased characteristic predicted value and a decreased estimate of uncertainty of prediction. In this case, the larger the characteristic quality value, the higher the possibility that a molecule that is actually generated exhibits an expected characteristic. That is, in molecular design, estimation processing with a characteristic quality value can be said to realize risk-avoiding estimation processing.

The characteristic quality value is not required to be a difference between a characteristic predicted value and a predetermined coefficient multiple of an estimate of uncertainty of prediction in a linear region. The characteristic quality value may be a value obtained by exponentiating one of a characteristic predicted value and an estimate of uncertainty of prediction a predetermined number of times, and calculating a difference between the obtained value and the other value, or a quotient of the obtained value and the other value. Therefore, when a characteristic predicted value which gives a smaller value with an increased possibility of having a desired characteristic, or with an increased degree of the characteristic is defined, the characteristic predicted value may be determined by division of the characteristic predicted value by an estimate of uncertainty of prediction, or other kinds of computation such as computation of these values in a log domain. Calculation of the characteristic quality value only requires derivation using a predicted value and uncertainty thereof, and there are no limitations on relevant functions, procedures and the like.

The candidate molecule identifier 111d identifies a molecule having a desired characteristic on the basis of the calculated characteristic quality value. As an example, in the candidate molecule identifier 111d, a molecule that gives a better characteristic quality value, among molecules whose characteristic predicted values are obtained in the characteristic predictor 111b, is more likely to be identified as a molecule having a desired characteristic. Here, the term "good characteristic quality value" means that a characteristic quality value indicating that the possibility of being predicted to have a desired characteristic, or the degree of the characteristic is high is obtained.

As another example, the candidate molecule identifier 111d may identify, as a molecule having a desired characteristic, a molecule, in which the characteristic quality value meets a predetermined condition, among molecules whose characteristic predicted values are calculated in the characteristic predictor 111b. As still another example, the candidate molecule identifier 111d may rearrange a plurality of molecules whose characteristic predicted values are calculated in the characteristic predictor 111b, with the molecules ranked on the basis of the characteristic quality value, and identify molecules within a predetermined range, among the rearranged molecules, as molecules having a desired characteristic. Here, the candidate molecule identifier 111d may identify a certain number of molecules as molecules having a desired characteristic such that molecules having a better characteristic quality value are given higher priority. The candidate molecule identifier 111d may identify, as a molecule having a desired characteristic, a molecule in which the characteristic predicted value and the estimate of uncertainty of prediction meet predetermined conditions, respectively.

The accuracy of the characteristic predicted value calculated by the characteristic predictor 111b influences identification of molecules. Depending on training data of characteristic prediction models, there is a difference in the accuracy of characteristic prediction based on sequence information. Here, the accuracy of prediction may be rephrased as reliability of prediction or certainty of prediction. Low accuracy of prediction can be rephrased as low reliability of prediction, low certainty of prediction, or high uncertainty of prediction. High accuracy of prediction can be rephrased as high reliability of prediction, high certainty of prediction, or low uncertainty of prediction.

In the molecular design device 1 of the present embodiment, not only a characteristic predicted value but also an estimate of uncertainty of prediction is used as an index for the processing to infer a molecule having a desired characteristic. That is, in the molecular design device 1 of the present embodiment, not a mathematical model for predicting a characteristic value, but a mathematical model for predicting a characteristic value and estimating uncertainty of prediction is used as a target to be used by the candidate molecule identifier in identification of a candidate molecule. Accordingly, a candidate molecule in which the characteristic predicted value is high, but certainty of the prediction is low can be prevented from being identified as a candidate molecule by the inference processing. That is, a molecule which is likely to fail to have a characteristic as high as expected when the molecule is actually generated is prevented from being identified. Accordingly, the burden accompanying drug discovery can be further reduced by the present embodiment.

(Second Embodiment)

Figure 2 shows a block diagram illustrating an example of a drug discovery system 100 comprising a molecular design device 1 according to a second embodiment. The following description mainly includes differences between the present embodiment and the first embodiment. The descriptions of the same points as in the first embodiment will be incorporated unless otherwise specified.

The molecular design device 1 according to the present invention is an example of an inference device for inferring a molecule which is expected to have a desired characteristic, from prediction information that will be described later.

The molecular design device 1 comprises an inferrer 111 comprising, for example, a sequence information processor 111a, a characteristic predictor 111b, a prediction information processor 111c, and a candidate molecule identifier 111d.

The inferrer 111 according to the present embodiment executes combination optimization algorithm (sometimes referred to simply as "combination optimization" in the present application) to infer a molecule that is expected to have a desired characteristic.

The sequence information processor 111a executes combination optimization to extract sequence information on any number of molecules from a preset search space. That is, the sequence information processor 111a repeats processing in which by combination optimization, sequence information on some molecules that are expected to have a desired characteristic is extracted from candidates of sequences of building blocks in the search space using an updated extraction parameter. The search space can be arbitrarily set. The search space may be set on the basis of, for example, training data for use in training of a prediction model for use in characteristic prediction. The number of times the sequence information is acquired, that is, the number of repetitions of acquisition of sequence information may be any natural number greater than or equal to 1. In the inferrer 111, the number of times the sequence information is acquired may be preset. The inferrer 111 may count, as the number of repetitions, the number of times the sequence information processor 111a actually extracts sequence information. In the present embodiment, sequence information on molecules contained in the search space can form an element of a sequence information collection.

The sequence information processor 111a outputs the extracted sequence information on a plurality of molecules to the characteristic predictor 111b.

The characteristic predictor 111b according to the present embodiment generates, on the basis of sequence information on each molecule which is input from the sequence information processor 111a, prediction information representing a characteristic of the molecule, using a prediction model, followed by output of the prediction information generated for each molecule to the prediction information processor 111c. The prediction model is generated by, for example, learning based on training data comprising a plurality of sets of sequence information on each molecule and a result of evaluating the characteristic of the molecule.

The prediction information on each molecule includes, for example, a characteristic predicted value of each molecule. Thus, the characteristic predictor 111b may comprise a predicted value calculator 111x configured to calculate a characteristic predicted value from sequence information on each molecule.

The prediction information on each molecule may include, for example, a characteristic predicted value of each molecule, and an estimate of uncertainty of the characteristic predicted value. The characteristic predictor 111b may comprise a prediction uncertainty estimator 111y configured to calculate an estimate of uncertainty of the characteristic predicted value. Accordingly, the prediction information on each molecule which is output from the characteristic predictor 111b can include both a characteristic predicted value of each molecule and an estimate of uncertainty of the characteristic predicted value.

The prediction information processor 111c acquires prediction information on each molecule from the characteristic predictor 111b. When the prediction information includes a characteristic predicted value and an evaluated value of uncertainty of the characteristic predicted value, the prediction information processor 111c may calculate a characteristic quality value on the basis of the characteristic predicted value of each molecule and the uncertainty of the characteristic predicted value with respect to sequence information on each molecule which is contained in the sequence information collection. In the present embodiment, the sequence information processor 111a uses, as prediction information, the characteristic quality value calculated by the prediction information processor 111c, to update an extraction parameter. Specific examples of the extraction parameter will be described later.

If the counted number of repetitions is smaller than the preset number of times the sequence information is acquired, the sequence information processor 111a updates the extraction parameter on the basis of the acquired prediction information. Therefore, in combination optimization, a series of processing including extraction of sequence information based on the extraction parameter after updating in the sequence information processor 111a, acquisition of prediction information based on the extracted sequence information by the characteristic predictor 111b, and updating of the extracted parameter by the sequence information processor 111a is repeated.

When the counted number of repetitions reaches the preset number of times the sequence information is acquired, the sequence information processor 111a stops processing with the combination optimization algorithm, and the prediction information processor 111c outputs the prediction information on each molecule, which is input from the characteristic predictor 111b, to the candidate molecule identifier 111d. The candidate molecule identifier 111d identifies, on the basis of the prediction information on each molecule which is input from the prediction information processor 111c, at least one candidate molecule from the sequence information collections at respective times. When the number of times the sequence information is acquired is N, the prediction information processor 111c identifies sequence information on at least one candidate molecule from N pieces of extracted sequence information on molecules. The candidate molecule identifier 111d may identify sequence information on at least one candidate molecule from N extracted collections of sequence information on molecules. When the prediction information includes a characteristic quality value, the candidate molecule identifier 111d can identify a candidate molecule on the basis of the characteristic quality value of each molecule.

The candidate molecule identifier 111d outputs sequence information on the identified candidate molecule.

In the present embodiment, Bayesian optimization can be applied in combination optimization, but the present embodiment is not limited thereto. Examples of the technique of Bayesian optimization include TPE (tree-structured Parzen estimator), and as a technique of evolutionary algorithm, any algorithm such as NSGA II (Elitist Non-dominated Sorting Genetic Algorithm) may be used.

In the present embodiment, by using combination optimization, a molecule having a desired characteristic can be inferred without predicting the characteristic values of all molecules contained in a search space with their sequence information. If a huge number of molecules are all covered as targets to be processed, it takes an enormous amount of calculation time, but by performing combination optimization on a search space as in the present embodiment, a molecule having a desired characteristic can be inferred without covering all the molecules. Accordingly, the burden accompanying drug discovery can be further reduced.

In the present embodiment, the molecular design device 1 may use, as an objective function for combination optimization, a mathematical model for estimating a characteristic value from sequence information, or a mathematical model for estimating a characteristic quality value. The output of the mathematical model is optimized by combination optimization. When a mathematical model for estimating a characteristic quality value is used, a molecule in which a characteristic indicated by a characteristic predicted value is preferred, but certainty of the prediction is low can be prevented from being identified as a candidate molecule. That is, it is possible to reduce the possibility that a molecule which is likely to fail to have a characteristic as high as expected when the molecule is actually generated is identified as a candidate molecule. Accordingly, the burden accompanying drug discovery can be further reduced.

Figure 3 shows an illustration for describing an example of sequence information collections in the first embodiment and the second embodiment. The "sequence ID" of Figure 3 is an identifier to identify sequence information on each molecule contained in the sequence information collection. Figure 3 shows sequence information on each molecule contained in the sequence information collection. The sequence information on each molecule represents a plurality of building blocks and arrangements for the respective building blocks. The arrangements of individual building blocks are shown between information D1 01 and information D102. For example, information D101 is information on building blocks related to position H1 in the sequences of molecules. VHL0001, VHL0002, VHL0003, VHL0004 and the like each represent a molecule in which the building block at position H1 is M.

Figure 4 shows a diagram illustrating an example of the hardware configuration of the molecular design device 1 according to the first embodiment and the second embodiment. The molecular design device 1 comprises a controller 11 comprising a processor 91 such as CPU and a memory 92 interconnected by a bus, and includes a computer system that executes a predetermined program. The computer system can also be considered to function as the molecular design device 1 comprising the controller 11, an input 12, a communicator 13, a storage 14 and an output 15 by execution of a program. In the present application, the phrase "execution of a program" or "executing a program" includes the meaning that processing indicated by each of one or more commands written in the program is executed.

More specifically, a processor 91 reads a program stored in a storage 14, and stores the read program in a memory 92. With the processor 91 executing the program stored in the memory 92, a function as the molecular design device 1 comprising the controller 11, an input 12, a communicator 13, a storage 14 and an output 15 is performed. The program is stored in, for example, the storage 14 in advance.

The controller 11 controls operations of various functional units of the molecular design device 1. The controller 11 has, for example, the function of the inferrer 111 that executes inference processing in which a molecule having a desired characteristic is inferred.

The input 12 comprises input devices such as a mouse, a keyboard and a touch panel. The input 12 may be constructed as an interface connecting the input devices to the molecular design device 1. The input 12 receives inputs of various kinds of information to the molecular design device 1. To the input 12, for example, training data of the prediction model is input.

The communicator 13 comprises a communication interface for connecting the molecular design device 1 to an external device. The communicator 13 communicates to the external device with or without wires. The external device is a device from which training data of the prediction model is sent. The external device may be a device to which sequence information on candidate molecules is sent. The molecular design device 1 may be connected to the internet, and realized as a server device capable of intercommunicating with each of one or more terminal devices.

The storage 14 is formed using a computer-readable storage medium device (non-transitory computer-readable recording medium) such as a magnetic hard disk or a semiconductor storage device. The storage 14 stores various kinds of information about the molecular design device 1. The storage 14 stores information input via, for example, the input 12 or the communicator 13. The storage 14 stores various kinds of information generated by processing executed by, for example, the controller 11. The storage 14 stores, for example, a prediction model, sequence information, and various parameters for use in combination optimization (which can include the above-described extraction parameter). The storage 14 stores, for example, the above-described program.

The output 15 outputs various kinds of information. The output 15 comprises a display device such as a CRT (cathode ray tube) display, a liquid crystal display, or an organic EL (electro-luminescence) display. The output 15 may be constructed as an interface connecting the display devices to the molecular design device 1. The output 15 outputs information input to, for example, the input 12 or the communicator 13. The output 15 may output various kinds of information generated by processing executed by, for example, the controller 11.

Figure 5 shows a diagram illustrating an example of the configuration of the controller 11 according to the first embodiment and the second embodiment. The controller 11 comprises the inferrer 111, an input controller 112, a communication controller 113, a storage controller 114, and an output controller 115. The inferrer 111 executes the inference processing. The input controller 112 controls operations of the input 12. The communication controller 113 controls operations of the communicator 13. The storage controller 114 controls operations of the storage 14. The output controller 115 controls operations of the output 15.

Next, an example of inference processing according to each embodiment will be described. Figure 6 shows a flowchart illustrating an example of a flow of processing executed by the molecular design device 1 in the first embodiment.

The sequence information processor 111a acquires a sequence information collection including sequence information on a plurality of molecules (step S101).

The characteristic predictor 111b calculates, with respect to each molecule forming the sequence information collection, calculates a characteristic predicted value, followed by calculation of an estimate of uncertainty of the characteristic value (step S102).

The candidate molecule identifier 111d identifies a candidate molecule on the basis of the characteristic predicted value calculated for each molecule, and the estimate of uncertainty of prediction (step S103). The technique for estimating uncertainty of prediction by the characteristic predictor 111b is not limited as long as it enables uncertainty quantification of the uncertainty. For example, a standard deviation of the characteristic predicted value may be calculated as the estimate of uncertainty of prediction. In the uncertainty quantification, conformal prediction may be used. The characteristic predictor 111b may divide learning data into a plurality of parts, calculate an error of prediction (prediction error) for each part, and quantify uncertainty on the basis of the distribution of the errors.

The candidate molecule identifier 111d outputs sequence information on the identified candidate molecule as sequence information on candidate molecules using the output 15 (step S104). Thereafter, the controller 11 completes the processing in Figure 6.

Before step S103, the prediction information processor 111c may calculate a characteristic quality value on the basis of the characteristic predicted value and the estimate of uncertainty of prediction which are obtained in step S102. The prediction information processor 111c may calculate a mean variance (MV) as an example of the characteristic quality value. MV is obtained by subtracting a penalty function g (x) from a product of a risk resistance parameter p and a characteristic predicted value f(x) with a predetermined prediction model (MV = pf(x) - g(x)). The risk resistance parameter is a positive real number value representing resistance to g(x) of uncertainty of the characteristic predicted value f(x), that is, reliability of the predicted value f(x). The prediction information processor 111c calculates a standard deviation as an example of an estimate g(x) of uncertainty. MV may be applied to portfolio optimization in financial engineering. In financial engineering, MV may be used as an index in which expected reward as a mean and variance of reward as a risk are integrated. MV is an index which can be utilized in various fields and for various purposes regardless of the optimization algorithm (see, for example, Q. Zhu and V. Y. F. Tan: Thompson Sampling Algorithms for Mean-Variance Bandits (2020 ICML), S. Takemori: Distributionally-Aware Kernelized Bandit Problems for Risk Aversion (2022 ICML)).

The prediction information processor 111c may execute calculation using, as an example of the characteristic quality value, uncertainty g(x) as a constraint condition. For example, the prediction information processor 111c may provide a threshold $\tau$ for uncertainty g(x), and set the characteristic quality value to, in the case where the uncertainty g(x) is equal to or smaller than $\tau$, a characteristic predicted value f'(x), and to, in the case where the uncertainty g(x) is larger than $\tau$, a sufficiently small value (for example, a minimum value that can be given by f(x)). This configuration is represented by expression (1).

[Expression 1]

$$\text{Characteristic quality value} = \begin{cases} f'(x) & if \quad g(x) \leq \tau \\ \min f'(x) & if \quad g(x) > \tau \end{cases} \quad ...(1)$$

The prediction information processor 111c may calculate a characteristic quality value on the basis of a plurality of physical property values. For example, the prediction information processor 111c provides a threshold $\tau 1$ for a first physical property value g1(x) and a threshold $\tau 2$ for a second physical property value g2(x). When the first physical property value g1(x) is smaller than $\tau 1$ and the second physical property value g2(x) is smaller than $\tau 2$, the prediction information processor 111c may set the characteristic quality value to the characteristic predicted value f(x). On the other hand, when the first physical property value g1(x) is equal to or larger than $\tau 1$ or the second physical property value g2(x) is equal to or larger than $\tau 2$, the prediction information processor 111c may set the characteristic quality value to a sufficiently small value (for example, a minimum possible value of f'(x)).

Alternatively, the prediction information processor 111c may calculate a characteristic quality value on the basis of a predicted value Pr of the probability of meeting the constraint ($x \in F|x$). That is, the prediction information processor 111c may multiply the characteristic predicted value f(x) with a predicted model by the predicted value of a probability of meeting the constraint, and sample only sequence information in which the probability of meeting the constraint is predicted to be high. In this example, the characteristic quality value approaches 0 when the probability of meeting the constraint is low, and the characteristic quality value approaches the objective index itself as the probability of meeting the constraint becomes higher (closer to 1). This characteristic quality value is represented by expression (2).

[Expression 2]

$$\text{Characteristic quality value} = f'(x) \cdot \Pr(x \in F \mid x) \quad ...(2)$$

The number of constraints may be 1, or may be 2 or more. For example, when a characteristic quality value based on a plurality of physical property values is calculated, the prediction information processor 111c may set a constraint condition on each physical property value, and multiply the characteristic predicted value f'(x) with a prediction model by a probability of meeting all the conditions. In this case, the probability of meeting all the conditions may be a product of probabilities of meeting the constraint conditions on the physical property values.

Figure 7 shows a flowchart illustrating an example of a flow of processing executed by the molecular design device 1 in the second embodiment.

The sequence information processor 111a acquires, as training data, a data set D including a plurality of data pairs each of which is a set of input value x versus measured value y (step S201). For example, the data set D input from the input 12 or the communicator 13 is stored in the storage 14 in advance. The sequence information processor 111a reads the data set D from the storage 14.

The sequence information processor 111a uses acquired training data to learn, for example, a prediction model for predicting input value versus measured value by executing Gaussian process regression (step S202).

The inferrer 111 executes combination optimization to identify a candidate molecule (step S203). The candidate molecule identifier 111d outputs sequence information on the identified candidate molecule using the output 15 (step S204). Thereafter, the processing in Figure 7 is completed.

<Example of Application to MBO>

Next, an example of application to MBO will be described.

In the present example of application, offline MBO which is a type of in-silico agent design methods and building block-based molecular design are combined and applied.

The offline MBO is a method for searching for an optimal molecule in a "delegate" prediction model generated from the acquired data. The method pertains to black box optimization involving a proxy model as a black box, and is also known as inverse analysis. Here, MBO is a technique in which a prediction model is learned using accumulated experimental data as training data in advance, an agent candidate molecule is evaluated on the basis of a characteristic value obtained using the prediction model obtained by the learning, and the prediction model is updated on the base of results of the evaluation. In the following description, the prediction model may be referred to as a proxy model.

In the present example of application, a discrete input value can be a target to be optimized by MBO. In MBO, it is assumed that discrete input value $x \in X$ versus measured value y is given as an unknown function f (x), a data set D including n sets of discrete input value x versus measured value y (where n is an integer of 2 or more) is given in advance, and additional input value x versus measured value y cannot be acquired. The discrete input value x versus measured value y gives a correct solution (also referred to as an oracle) of the function f(x). General MBO is designed to determine an input value x which maximizes the function f(x) is in a discrete value space X. The above-described technique can be considered as a problem of determining an input value x which maximizes a characteristic quality value $f(x) - \lambda g(x)$ instead of the function f(x). Here, a sequence is applied as the input x, and the function f(x) corresponds to a function for predicting a real number value corresponding to a characteristic value. The penalty function g(x) is a function that gives a real number value corresponding to uncertainty of the value of the function f(x). $\lambda$ corresponds to the coefficient $\alpha$. The input value x is expressed using a vectorial representation.

In offline MBO, the sequence information processor 111a is made to learn in advance using, as training data, the data set D including a plurality of sets of discrete input value x versus measured value y. In learning of the prediction model, the sequence information processor 111a determines a parameter of the prediction model such that a function value of an objective function for the discrete input value x is a predicted value y', and an index value representing a magnitude of a difference between the predicted value y' and the measured value y for the discrete input value x is minimized. In the present example of application, as described below, the inferrer 111 samples the discrete input value x, and updates the parameter of combination maximization on the basis of prediction results obtained using the prediction model for each sample.

The inferrer 111 can use, for example, the TPE in combination optimization. TPE is a black box optimization algorithm in which Parzen window density estimation is applied in combination with Bayesian optimization. TPE can accommodate a category parameter, and therefore can be applied to building block-based molecular design.

In the present example of application, the inferrer 111 implements the function of TPE, and is applied in molecular design as follows. TPE is a technique which is oriented to maximize the expected improvement (EI) of an objective function. First, the sequence information processor 111a applies a building block combination forming an agent candidate molecule to a search space, and assigns an output from the prediction model to an objective score collection Y corresponding to an input molecule collection X sampled from the search space. For example, the sequence information processor 111a sets a characteristic predicted value obtained with the prediction model as an objective score of TPE. The sequence information processor 111a samples the input molecule collection X from the search space, that is, assigns a candidate of a building block to each modification position in the building block combination. For example, when the agent candidate molecule is a protein, a candidate amino acid is assigned to each modification position in the sequence of the protein. As an example, Optuna implementation can be used for executing a TPE sampler.

Next, the characteristic predictor 111b calculates an estimate of the objective function using the prediction model on the input value determined by sampling (evaluation).

The prediction information processor 111c outputs the selected input value and the calculated estimate to the sequence information processor 111a, and the sequence information processor 111a updates the parameter of the optimization algorithm using the input value and the estimate input from the prediction information processor 111c. Here, the sequence information processor 111a assigns the selected input value and the calculated estimate to the input molecule collection X sampled from the search space, and the objective score collection Y, respectively. The inferrer 111 repeats the processes of data division, sampling, evaluation and updating, so that the results of evaluation of the input value obtained by sampling (sample value) are incorporated in the prediction model, and a probabilistic search for a combination of building blocks which is larger in estimate of the objective function is made.

Figure 8 illustrates an example of a flow of combination optimization processing executed by the molecular design device 1 according to the present example of application.

The inferrer 111 sets the initial value 0 of the number of times the sequence information is acquired. A loop R20 includes processing in steps S203a to S203c. The controller 11 defines that the condition for executing the loop R20 is that the number of repetitions is equal to or smaller than a predetermined number of times of sampling N. The number of times of sampling corresponds to the number of times the sequence information is acquired.

The sequence information processor 111a acquires sequence information on a plurality of molecules in accordance with the algorithm of combination optimization (step S203a). For example, when TPE is used as the algorithm of combination optimization, the sequence information processor 111a divides the collection of input values into two sets on the basis of the estimate obtained using the prediction model on each input value (corresponding to the characteristic predicted value) and a predetermined threshold y. One set (referred to as a "first set") comprises input values at which the estimate is equal to or larger than the threshold $\gamma$. The other set (referred to as a "second set") comprises input values at which the estimate is less than the threshold $\gamma$. The sequence information processor 111a samples input values which maximize the expected improvement of the objective function. The expected improvement corresponds to an amount of increase in expected value of the objective function before and after updating, and is known to be proportional to $p(x|y1)/p(x|y2)$ belonging to the first set with respect to the input value x. $p(x|y1)$ represents a density distribution of the input value x in the first set. $p(x|y2)$ represents a density distribution of the input value x in the second set. That is, EI is an example of the extraction parameter. The sequence information processor 111a samples input values which maximize the calculated EI.

The characteristic predictor 111b performs sequence evaluation with the prediction model. Here, the characteristic predictor 111b calculates, using the prediction model, estimates of the objective function for the sampled input values (step S203b).

The sequence information processor 111a assigns the selected input value and the calculated estimate to the input molecule collection X sampled from the search space, and the objective score collection Y. Therefore, a new input value joins the input molecule collection X matched against the objective function and sampled from the search space, and thus, the extraction parameter of the combination optimization algorithm involved in extraction of a sequence information collection is updated (step S203c).

The inferrer 111 updates the number of repetitions by adding 1 (increment) each time the processing in steps S203a to S203c is completed. The inferrer 111 repeats the processing in steps S203a to S203c when the number of repetitions is equal to or smaller than the number of times of sampling N.

The inferrer 111 completes the processing of loop R20 when the number of repetitions exceeds the number of times of sampling N. The candidate molecule identifier 111d may output, as sequence information on candidate molecules, a predetermined number of building block combinations in descending order of the estimate of the objective function, using the output 15. Thereafter, the processing in Figure 8 is completed.

Figure 9 illustrates another example of a flow of combination optimization processing executed by the molecular design device 1 according to the present example of application. The processing in Figure 9 is the same as the processing in Figure 8 in that the processing in steps S203a to S203c is repeated, while being different from the processing in Figure 8 in that step S203d and step S203e are provided instead of the loop R20. In the step S203d, the inferrer 111 sets the number of times of sampling N and the initial value 0 of the number of repetitions. After the processing in step S203b, the process proceeds to step S203e. In step S203e, the inferrer 111 adds 1 to the number of repetitions at this point, and determines whether the number of repetitions has reached N or not. When it is determined that the number of repetitions has reached N (step S203e YES), the processing in Figure 9 is completed. When it is determined that the number of repetitions has not reached N (step S203e NO), the process proceeds to step S203c. After the processing in step S203c, the process proceeds to step S203a. Thus, the processing in Figure 9 is different from the processing in Figure 8 in that the inferrer 111 does not update the extraction parameter when the number of repetitions exceeds the number of times of sampling N.

In examples of Figures 8 and 9, it is assumed that the completion condition is set as a time when the number of repetitions exceeds the number of times the sequence information is acquired, but the completion condition is not necessarily limited thereto. For example, the completion condition may be such that a target value of the estimate of the objective function is preset, and the estimate of the objective function reaches the target value.

In the verification example in the present application, TPE in which MV calculated from a predicted mean value and a predicted variance value of Gaussian process regression is used as an objective function may be referred to as MV-TPE. TPE in which a predicted mean of Gaussian process regression is used as an objective function may be referred to as Mean-TPE. In the present application, the predicted mean of the Gaussian process regression as an objective function refers to a predicted value itself.

<First Verification Example>

Next, verification examples in the present embodiment will be described. In a first verification example, the safety of the present example of application was verified using a GFP data set as training data of the prediction model. The GFP data set includes information on 56,086 GFP (green fluorescent protein) sequences and luminance as a characteristic thereof. GFP is a sample which is widely used in medical and biological studies. Generation of light GFP may be used as a benchmark for model-based optimization performance as a research target. The input value representing each GFP sequence is expressed using a vectorial representation of 756 dimensions. In the present verification, a tasks assessing protein embeddings method (TAPE) was used as a protein embedding model in acquisition of a vectorial representation.

As training data, a prediction model was learned using a Gaussian process (GP) on the basis of a GFP sequence in which the number of mutations from a parent sequence avGFP (aequorea victoria GFP) is 2 or fewer. In the following description, the prediction model obtained through the learning may be referred to as a proxy model. The parent sequence avGFP may be referred to simply as a parent sequence or template GFP. By this procedure, a sequence in which few of residues from a parent sequence are substituted is verified, and thus, a practical molecule optimization process is realized. In the present verification, a GFP sequence with 2 or fewer residue substitutions (edit distances) as illustrated in Figure 10 was adopted. As a pseudo-correction solution model, LightGBM (light gradient boosting machine) was learned using all data of the GFP data set. LightGBM may be used for ranking, classification and other tasks based on a decision tree algorithm. In such a configuration, GFP sequences around a parent sequence are covered by the proxy model, and a wider GFP space is covered as a search space by the pseudo-correct solution model.

The search space was defined as mutations in top 100 sequences in the training data. The search space includes 2 to 5 amino acid candidates at each of 37 mutation candidate positions as shown in Figure 17. The TPE parameter was set to "number of times of sampling: 3,000" and "multivariable: none" (i.e., the objective function is one variable) as shown in Figure 18. Top 10 sequences in the training data were used for warm-start initiation in each of MV-TPE and Mean-TPE.

Commonly, in agent discovery, 10 to 100 sequences are evaluated in one batch. In the present verification, as illustrated in Figure 10, top 96 sequences that were higher in score, i.e., estimate of the objective function when the pseudo-correct solution model was used were taken as proposed sequences, and used as targets to be evaluated.

In the verification, the processing in Figures 7 and 8 were executed using MV and the mean, respectively, as objective functions. The mean was used as a comparative example. MV and the mean each include characteristics. Among the characteristics, the luminance of GFP was used. Figure 11 shows a TPE-optimized trajectory, where a mean as an estimate of the objective function is shown in the upper part, and MV is shown in the lower part. In each part, the results of 10 times of optimization processing are shown. The broken line represents an estimate for the sample in each process.

Figure 12 shows a relationship between a mean (GP Mean) and a standard deviation (GP Std) of sample values in the processes. The use of MV as the objective function leads to a smaller standard deviation as compared to a case where the mean is used. This means that the use of MV reduces the uncertainty of an estimate.

Figure 13 shows a density distribution of edit distances of the samples with template GFP as a reference. The use of MV as the objective function leads to a smaller edit distance as a whole as compared to a case where the mean is used. This means that by utilizing MV, GFP having few mutations from template GFP is selected as a sample.

Figure 14 shows a distribution of score of proposed sequences obtained using a pseudo-correct solution model. The score represents an estimate of the luminance of fluorescence emitted by GFP. The use of MV as the objective function leads to a higher luminance as compared to a case where the mean is used.

Figure 15 shows an edit distances of a proposed sequence with template GFP as a reference. The use of MV as the objective function leads to a smaller edit distance as compared to a case where the mean is used. This means that by using MV, sequences having few mutations are sampled, that is, safe optimization can be realized.

Figure 16 shows a standard deviation (GP Std) of a proposed sequence. The use of MV as the objective function leads to a smaller standard deviation as compared to a case where the mean is used. This demonstrates that by using MV, sequences with little uncertainty of the luminance as a characteristic value are sampled, so that safe optimization can be realized.

<Second Verification Example>

Next, a second verification example will be described. In the second verification example, data about bispecific antibodies was used as training data, and the Gaussian process was executed to learn the prediction model. The training data used in the present verification example is data representing the sequences of bispecific antibodies which recognize MarvelD3 and CD3 as antigens, and the characteristic values of binding ability. An octet value was used as the characteristic value of binding ability. A vectorial representation obtained using TAPE was used as an input value to the prediction model. A sequence of a bispecific antibody protein used as a sample is represented by the vectorial representation.

MarvelD3 is a tight junction protein having 4-transmembrane structure. In the present verification example, MarvelD3 was set as a target candidate for an anticancer drug. The development of a bispecific antibody that bridges a cancer antigen and an antigen on T cells is expected the application to cancer treatment. In the present verification, the learned prediction model was used when a candidate of an anti-MarvelD3 having a better characteristic was determined from lead antibodies.

In the present verification, a plurality of measurements of the octet value of an antibody sequence were made, and the antibody sequence and the octet value of the antibody sequence, which had been obtained by the measurement, were used as inputs to the prediction model. By batch measurement, the octet values typically of 100 or fewer antibody sequences are acquired in one measurement.

Here, an antibody used in the measurement was acquired by the following procedure. First, a plasmid encoding a heavy chain or a light chain designed in advance was provided, and a recombinant antibody was transiently expressed using Expi293F cells. The antibody was taken from a culture supernatant using protein A, and dissolved in a buffer solution. The dissolving buffer solutions were mixed under reducing conditions to prepare a MarvelD3/CD3 bispecific antibody. Here, charge repulsion was applied between identical heavy chains to perform selective heavy chain heterodimerization. The concentration of the antibody in the buffer solution was determined by an absorbance at 280 nm. Thereafter, ion-exchange chromatography was performed on the buffer solution containing a bispecific antibody, and it was confirmed that an intended MarvelD3/CD3 antibody was provided.

An Octet HTX system was used in measurement of the octet value. Extracellular vesicles having a CD81 protein and a human MarvelD3 on surfaces thereof were caught on a sensor chip using an anti-CD81 antibody. After a 600-second baseline step in a D-PBS(-) solution containing 0.1% BSA, an association response and a dissociation response in identical buffer solutions containing 20 nM of the antibody were measured for 900 seconds and 1,500 seconds, respectively. The binding ability of the antibody appears as a wavelength change between the baseline step and the end of the association stage. The measurement was performed with vibrations made at a temperature of 30°C and a vibration rate of 1,000 times per minute in the stages of the baseline step, association and dissociation.

In optimization of the prediction model, MV-TPE and Mean-TPE were each executed as in the first verification example. In the present verification, among candidate sequences obtained by sampling, top 48 sequences in terms of the estimate of the objective function were evaluated as proposed sequences.

Figure 19 shows distributions of mean values and standard deviations of estimates obtained by sampling in each process, for MV-TPE and Mean-TPE, respectively. The standard deviation represents uncertainty, and the higher the mean value of the estimate, the better the octet value. According to Figure 19, the mode value of the standard deviation is 1.0 when the mean (Mean) is used as the objective function, whereas the mode value of the standard deviation is 0.3 when MV is used as the objective function. The mode value of the mean is 2.3 when the mean is used as the objective function, whereas the mode value of the mean is 1.5 when MV is used. These results show that the uncertainty of the octet value as a characteristic predicted value is higher when the mean is used than when MV is used as the objective function.

Figure 20 shows a t-SNE (t-distributed stochastic neighbor embedding) visualization diagram of sequences obtained by sampling. The t-SNE is a technique in which high-dimensional data is compressed to a low dimension for visualization. In Figure 20, distributions of TAPE-based vectorial representations representing individual sequences are shown on a two-dimensional plane for training data (Train), the mean (Mean) and MV, respectively. According to Figure 20, sequences sampled with the use of the mean as the objective function are widely different from the training data, whereas sequences sampled with the use of MV are closer to the training data. This shows that it is possible to search for safer regions while suppressing uncertainty to a higher degree by MV-TPE than by Mean-TPE, and that MV-TPE enables avoidance of pathologic samples.

Figure 21 shows mean values (GP Mean) of estimates of octet values of the 48 sequences to be evaluated when the objective functions of TPE are the mean (Mean) and MV, respectively. According to Figure 21, the mean value of octet values obtained using MV was lower than the mean value of octet values obtained using the mean as the objective function.

Figure 22 shows standard deviations (GP Std) of the estimates for the mean (Mean) and MV, respectively.

According to Figure 22, the standard deviation of octet values obtained using MV was lower than the standard deviation of octet values obtained using the mean as the objective function. From the results, it is presumed that for sequences sampled by MV, stable expression and binding ability are unlikely to deteriorate because of low uncertainty.

Figure 23 shows distributions of expression levels of the 48 sequences to be evaluated for the mean (Mean) and MV, respectively. According to Figure 23, when the mean was used as the objective function, the expression levels of top 48 sequences were extremely low, and a sufficient amount of samples to measure the octet value were not obtained. On the other hand, when MV is used as the objective function, almost all of top 48 sequences are significantly expressed.

Figure 24 shows distributions of octet values of sequences for MV and training data, respectively. MV and training data are similar in tendency of distribution of octet values. This shows that the use of MV as the objective function enables acquisition of sequences comparable in binding ability to training data, and hence a parent sequence. Further, by MV, several sequences having an octet value higher than the maximum value in training data were obtained.

<Conclusions>

The verification examples described above have shown that offline model-based optimization (MBO) according to the present example of application can be applied to building block-based molecular design which is a common practice in agent design, thereby realizing in-silico implementation (that is, implementation via a calculator). Here, the tree-structured Parzen estimator (TPE) which is a technique for Bayesian optimization can be applied to building block-based molecular design pertaining to optimization of a combination of category variables. It has also been shown that when offline MBO is applied, MV used in financial engineering contributes to search of safer sequences by avoiding pathologic behaviors. That is, by incorporating risk averse prediction in MBO for molecular design, overestimation of extrapolation regions in prediction of a characteristic of a candidate molecule can be prevented to prevent proposition of a molecule that is low in prediction reliability. By the verification examples described above, the present embodiment has been shown to be useful for searching for safe sequences in design of therapeutic antibodies.

The molecular design device 1 of the embodiment which is configured as described above performs Bayesian optimization using, as an objective function, a model configured to obtain a characteristic quality value on the basis of agent candidate molecular sequence information. Therefore, the burden accompanying drug discovery can be further reduced.

(Modifications)

The molecular design device 1 may be assembled using a plurality of information processing devices connected communicably via a network. In this case, the functional units of the molecular design device 1 may be dispersed among a plurality of information processing devices. The drug discovery system 100 may comprise a plant (not shown) that generates an optimal molecule estimated by the molecular design device 1. The molecular design device 1 outputs, to the plant, output information representing a biological sequence of an optimal molecule estimated by the technique described above. The plant carries out the step of generating a molecular compound having the biological sequence indicated by the output information input from the molecular design device 1.

All or a part of the functions of the molecular design device 1 may be realized using hardware such as ASIC (Application Specific integrated Circuit), PLD (Programmable Logic Device), or FPGA (Field Programmable Gate Array). The program may be recorded in a computer-readable recording medium. The "computer-readable storage medium" is, for example, a storage device such as a portable medium, such as a flexible disk, a magneto-optical disk, a ROM, and a CD-ROM, and a hard disk built in computer systems. The program may be sent via a telecommunication line.

The molecular design device 1 is an example of an estimation device. The function may be realized by another type of equipment such as a device whose main function is not molecular design, or information equipment comprising a common computer system.

For example, the present embodiment may be realized as a system comprising a processor and a memory. In the system, the memory is configured to store one or more commands, and the commands may be commands for casing the processor to execute a procedure of calculating, with respect to a molecule forming an element of a sequence information collection which is a collection of sequence information on a plurality of different molecules, a characteristic predicted value of the molecule using a prediction model for predicting a characteristic of a molecule from sequence information on the molecule, followed by estimation of uncertainty of the prediction, and a procedure of searching for a molecule having a more desired characteristic on the basis of the characteristic predicted value and the uncertainty of prediction.

The commands may be commands for causing the processor to execute an acquisition procedure of acquiring, in accordance with a combination optimization algorithm, a sequence information collection which is a collection of sequence information on a plurality of different molecules, a prediction procedure of calculating, using a prediction model for predicting a characteristic of a molecule from sequence information on the molecule, a characteristic predicted value of a molecule forming an element of the sequence information collection, an updating procedure of updating an extraction parameter of the combination optimization algorithm on the basis of the characteristic predicted value of each molecule so as to include a molecule having a more desired characteristic, and a searching procedure of searching for a molecule having a more desired characteristic on the basis of the characteristic predicted value.

The present embodiment may be realized as a non-transitory computer-readable medium for storing one or more commands. The commands may be commands for causing a computer to execute a procedure of calculating, with respect to a molecule forming an element of a sequence information collection which is a collection of sequence information on a plurality of different molecules, a characteristic predicted value of the molecule using a prediction model for predicting a characteristic of a molecule from sequence information on the molecule, followed by estimation of uncertainty of the prediction, and a procedure of searching for a candidate of a molecule having a more desired characteristic on the basis of the characteristic predicted value and the uncertainty of prediction. The commands may be commands for causing the computer to execute an acquisition procedure of acquiring, in accordance with a combination optimization algorithm, a sequence information collection which is a collection of sequence information on a plurality of different molecules, a prediction procedure of calculating, using a prediction model for predicting a characteristic of a molecule from sequence information on the molecule, a characteristic predicted value of a molecule forming an element of the sequence information collection, an updating procedure of updating an extraction parameter of the combination optimization algorithm on the basis of the characteristic predicted value of each molecule so as to include a molecule having a more desired characteristic, and a searching procedure of searching for a molecule having a more desired characteristic on the basis of the characteristic predicted value.

The present embodiment may be realized as a computer-implemented method using an artificial intelligence engine. The computer-implemented method is processing of a candidate collection which is a collection having, as an element, sequence information on agent candidate molecules which is information on sequences of units constituting the agent candidate molecules, the method comprising a control step of estimating an optimal molecule which is an agent candidate molecule giving an optimal value of a desired characteristic, among the agent candidate molecules represented by respective elements of the candidate collection, by executing combination optimization, and a step of outputting information about the optimal molecule. An objective function of the combination optimization is a model for executing a mathematical model for estimating a characteristic value of a desired characteristic on the basis of the sequence information on agent candidate molecules and acquiring uncertainty of a result of the estimation, and acquiring a characteristic quality value calculated on the basis of the determination of the characteristic value and the uncertainty.

While some embodiments are described above, these embodiments are presented as examples, and are not intended to limit the scope of the invention. These embodiments can be carried out in various other forms, and various omissions, replacements and changes can be made without departing from the spirit of the invention. These embodiments and modifications thereof are within the inventions set forth in claims and equivalents thereof as they are within the scope and the spirit of the invention. Documents cited herein are incorporated herein by reference.

[Reference Signs List]

1...molecular design device, 11...controller, 12...input, 13...communicator, 14...storage, 15...output, 100...drug discovery system, 111...inferrer, 112...input controller, 113...communication controller, 114...storage controller, 115...output controller, 91...processor, 92...memory

## Claims

1. An information processing system for identifying a molecule suitable for a drug candidate, comprising:

   a characteristic predictor configured to calculate, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and
   a candidate molecule identifier configured to search for a candidate of a molecule having a desired characteristic on the basis of the characteristic predicted value and an estimate of the uncertainty of prediction.

2. The information processing system according to claim 1, further comprising a prediction information processor configured to calculate a characteristic quality value on the basis of the characteristic predicted value and the estimate of uncertainty of prediction,
   wherein the candidate molecule identifier identifies at least one candidate molecule from elements of the building block combination information collection on the basis of the characteristic quality value.

3. The information processing system according to claim 2, wherein the characteristic quality value is an output value of a predetermined function in which two variables, one of which is the characteristic predicted value and the other of which is the estimate of uncertainty of prediction, are inputs.

4. The information processing system according to claim 2 or 3, wherein the prediction information processor calculates a mean variance as an objective function that gives the characteristic quality value.

5. The information processing system according to any one of claims 2 to 4, wherein the characteristic quality value increases with an increase in the characteristic predicted value, and decreases with a decrease in the estimate of uncertainty of prediction.

6. The information processing system according to any one of claims 2 to 5, comprising a building block combination information processor configured to acquire the building block combination information collection on the basis of the characteristic predicted value of the molecule or the characteristic quality value using a combination optimization algorithm.

7. The information processing system according to any one of claims 2 to 6, wherein the characteristic quality value increases with an increase in the characteristic predicted value, and decreases with a decrease in the estimate of uncertainty of prediction, the building block combination information collection is acquired using a combination optimization algorithm, and an extraction parameter of the combination optimization algorithm is updated on the basis of the characteristic predicted value of the molecule or the characteristic quality value.

8. The information processing system according to claim 7, wherein the combination optimization algorithm is a tree-structured Parzen estimator.

9. The information processing system according to any one of claims 1 to 8, wherein the prediction model is a prediction model generated by learning based on building block combination information on a plurality of molecules for training and results of evaluating the characteristics of the molecules.

10. The information processing system according to any one of claims 1 to 9, wherein the building block combination information is sequence information on the molecule.

11. The information processing system according to any one of claims 1 to 10, wherein the molecule is at least one of a nucleic acid, a peptide, a cyclic peptide, a protein, an antibody, and a low-molecular compound.

12. The information processing system according to any one of claims 1 to 11, wherein the characteristic is at least one of binding ability, pharmacological activity, a physical property, kinetics, and safety.

13. The information processing system according to any one of claims 1 to 12, wherein the molecule is a molecule that binds to a target molecule, and the characteristic is ability to bind to the target molecule.

14. An information processing method in an information processing system for identifying a molecule suitable for a drug candidate, the method comprising the steps of:

calculating, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and

searching for a candidate of a molecule having a more desired characteristic on the basis of the characteristic predicted value and the uncertainty of prediction.

15. A program configured to cause a computer to execute:

a procedure of calculating, with respect to a molecule forming an element of a building block combination information collection which is a collection of building block combination information on a plurality of different molecules, a characteristic predicted value of the molecule using a prediction model for predicting a characteristic of a molecule from building block combination information on the molecule, followed by estimation of uncertainty of the prediction; and

a procedure of searching for a candidate of a molecule having a more desired characteristic on the basis of the characteristic predicted value and the uncertainty of prediction.

**16.** A method for producing a molecular compound, the method comprising:

an input step of accessing a building block combination information collection which is a collection of building block combination information on a plurality of different molecules, and
inputting the building block combination information collection to a prediction model;
an inference step of searching for a molecule having a more desired characteristic from the building block combination collection, on the basis of a characteristic predicted value and an estimate of uncertainty of prediction for each molecule which are contained in the building block combination information collection and output from the prediction model, followed by identification as a candidate molecule;
an output step of outputting building block combination information on the candidate molecule; and
a generation step of generating the molecular compound having a molecular sequence represented by the building block combination information.

**17.** An information processing system for identifying a molecule suitable for a drug candidate, comprising:

a building block combination information processor configured to acquire, in accordance with a combination optimization algorithm, a building block combination information collection which is a collection of building block combination information on a plurality of different molecules;
a characteristic predictor configured to calculate, with respect to a molecule forming an element of the building block combination information collection, a characteristic predicted value using a prediction model for predicting a characteristic of a molecule from building block combination information on the molecule; and
a candidate molecule identifier configured to search for a candidate of a molecule having a desired characteristic on the basis of the characteristic predicted value,
wherein the building block combination information processor
updates an extraction parameter of the combination optimization algorithm on the basis of the characteristic predicted value of the molecule so as to include a molecule having a more desired characteristic.

**18.** An information processing method in an information processing system for identifying a molecule suitable for a drug candidate, the method comprising the steps of:

acquiring, in accordance with a combination optimization algorithm, a building block combination information collection which is a collection of building block combination information on a plurality of different molecules;
calculating, with respect to a molecule forming an element of the building block combination information collection, a characteristic predicted value of the molecule using a prediction model for predicting a characteristic of a molecule from building block combination information on the molecule;
searching for a molecule having a more desired characteristic on the basis of the characteristic predicted value; and
updating an extraction parameter of the combination optimization algorithm on the basis of the characteristic predicted value of the molecule so as to include a molecule having a more desired characteristic.

**19.** A program configured to cause a computer to execute:

an acquisition procedure of acquiring, in accordance with a combination optimization algorithm, a building block combination information collection which is a collection of building block combination information on a plurality of different molecules;
a prediction procedure of calculating, using a prediction model for predicting a characteristic of a molecule from building block combination information on the molecule, a characteristic predicted value of a molecule forming an element of the building block combination information collection;
an updating procedure of updating an extraction parameter of the combination optimization algorithm on the basis of the characteristic predicted value of each molecule so as to include a molecule having a more desired characteristic; and
a searching procedure of searching for a molecule having a desired characteristic on the basis of the characteristic predicted value.

**20.** A method for producing a molecular compound, the method comprising:

an acquisition step of acquiring, in accordance with a combination optimization algorithm, a building block combination information collection which is a collection of building block combination information on a plurality of different molecules;
an input step of inputting the building block combination information collection to a prediction model;
an updating step of updating, on the basis of a characteristic predicted value of each molecule which is contained in the building block combination information collection and output from the prediction model, an extraction parameter of the combination optimization algorithm so as to include a molecule having a more desired characteristic;
a step of searching for a molecule having a more desired characteristic from the building block combination information collection, on the basis of the characteristic predicted value, followed by identification as a candidate molecule;
an output step of outputting building block combination information on the candidate molecule; and
a generation step of generating the molecular compound having a molecular sequence represented by the building block combination information.

Fig.1

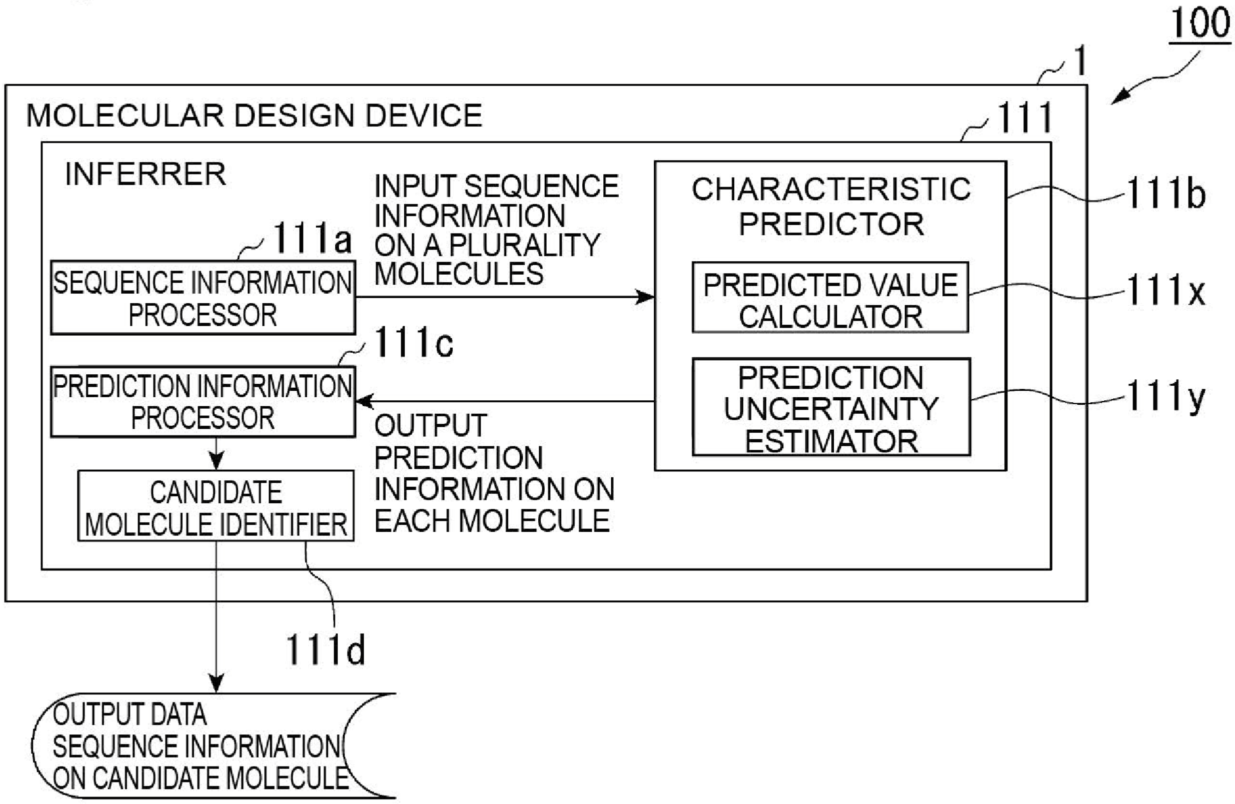
100
1
MOLECULAR DESIGN DEVICE
111
INFERRER
INPUT SEQUENCE INFORMATION ON A PLURALITY MOLECULES
CHARACTERISTIC PREDICTOR
111b
111a
SEQUENCE INFORMATION PROCESSOR
PREDICTED VALUE CALCULATOR
111x
111c
PREDICTION INFORMATION PROCESSOR
PREDICTION UNCERTAINTY ESTIMATOR
111y
OUTPUT PREDICTION INFORMATION ON EACH MOLECULE
CANDIDATE MOLECULE IDENTIFIER
111d
OUTPUT DATA SEQUENCE INFORMATION ON CANDIDATE MOLECULE

# Fig.2

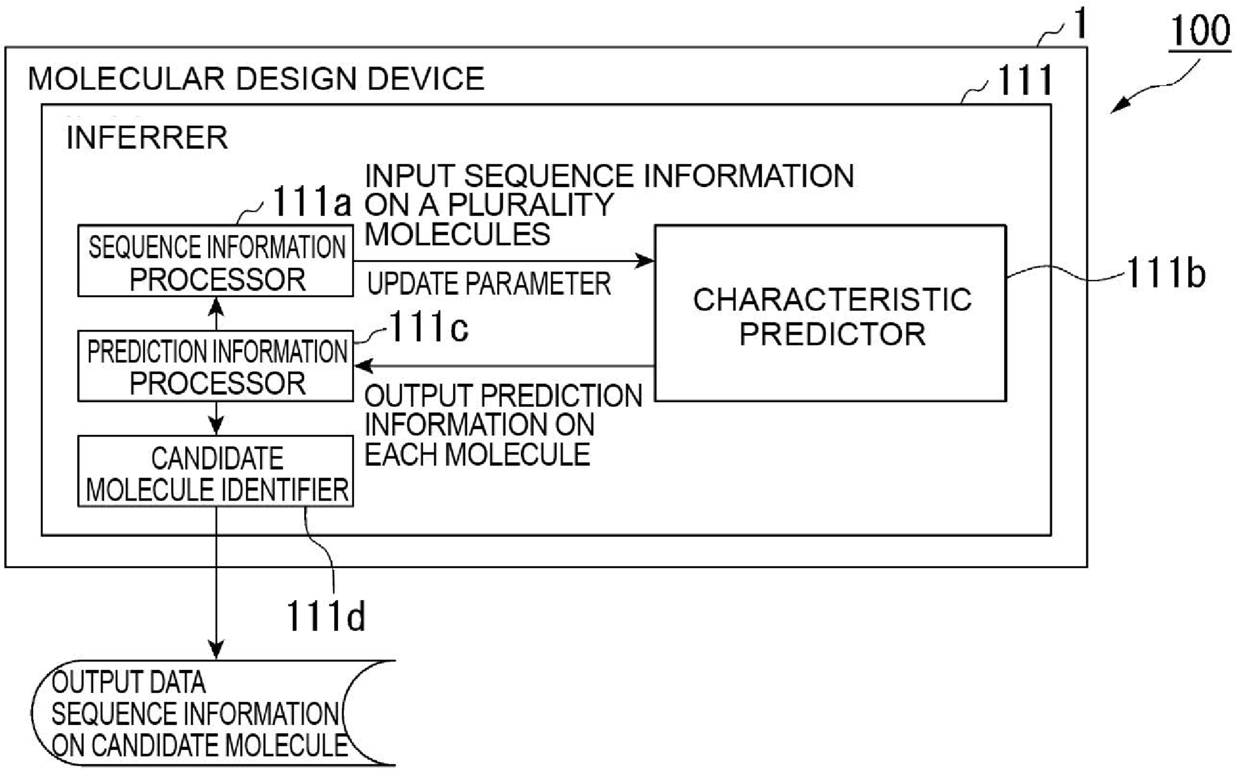
1
100
MOLECULAR DESIGN DEVICE
111
INFERRER
INPUT SEQUENCE INFORMATION ON A PLURALITY MOLECULES
111a
SEQUENCE INFORMATION PROCESSOR
UPDATE PARAMETER
CHARACTERISTIC PREDICTOR
111b
PREDICTION INFORMATION PROCESSOR
111c
OUTPUT PREDICTION INFORMATION ON EACH MOLECULE
CANDIDATE MOLECULE IDENTIFIER
111d
OUTPUT DATA SEQUENCE INFORMATION ON CANDIDATE MOLECULE

*Fig.3*

D101 … D102

| SEQUENCE ID | H1 | H2 | … | H35a | H35b | H36 | … | L1 | L2 | … | L27 | … |
|---|---|---|---|---|---|---|---|---|---|---|---|---|
| VHL0001 | M | E | … | P | S | Q | … | M | F | … | A | … |
| VHL0002 | M | E | … | D | S | Q | … | M | F | … | R | … |
| VHL0003 | M | E | … | W | S | Q | … | M | F | … | G | … |
| VHL0004 | M | E | … | P | S | R | … | M | F | … | H | … |
| … | … | … | … | … | … | … | … | … | … | … | … | … |

*Fig.4*

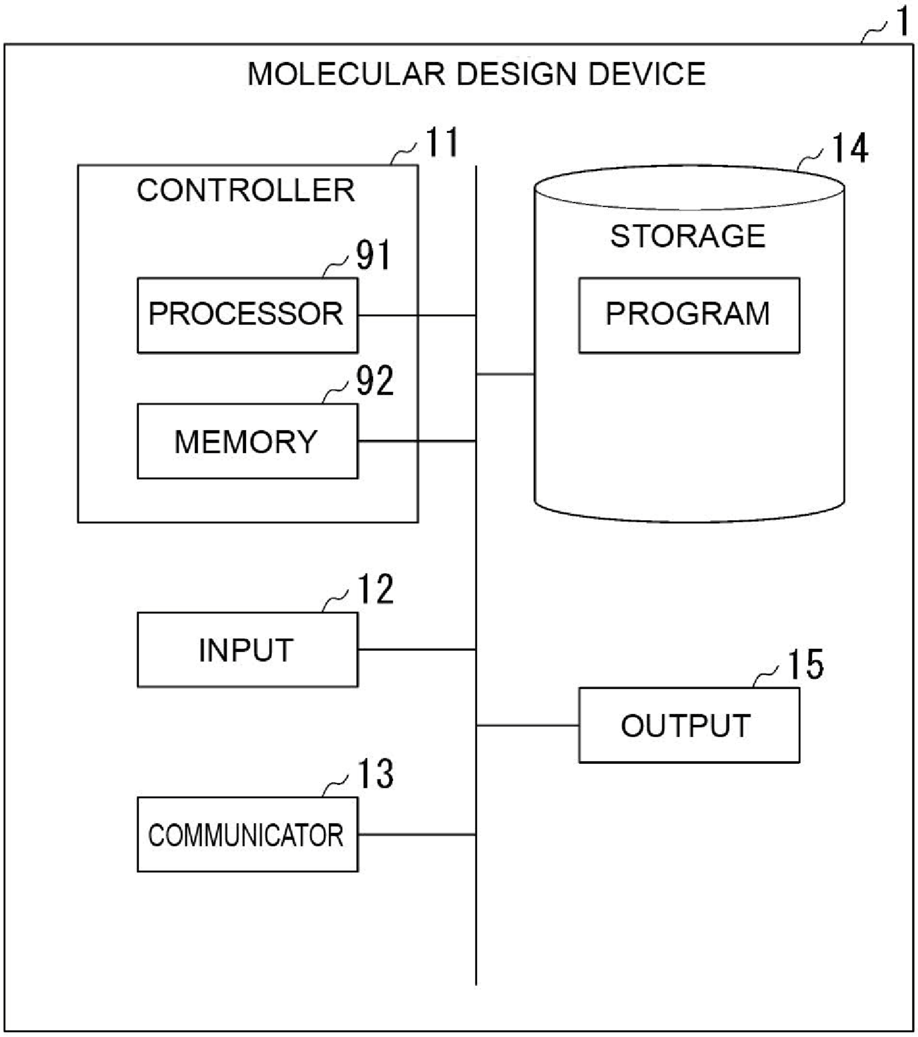
1
MOLECULAR DESIGN DEVICE
11
CONTROLLER
91
PROCESSOR
92
MEMORY
14
STORAGE
PROGRAM
12
INPUT
15
OUTPUT
13
COMMUNICATOR

Fig.5

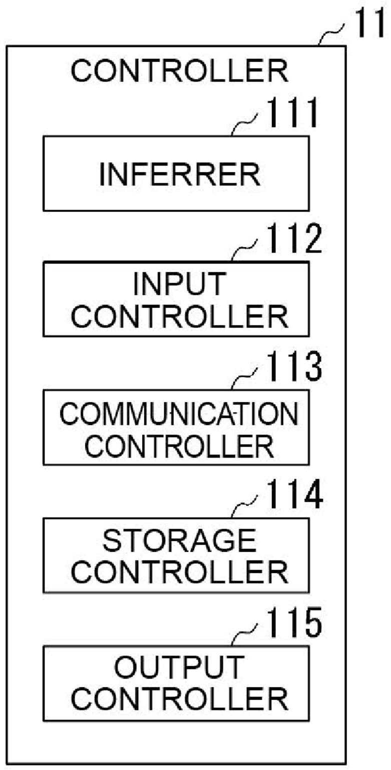
11
CONTROLLER
111
INFERRER
112
INPUT CONTROLLER
113
COMMUNICATION CONTROLLER
114
STORAGE CONTROLLER
115
OUTPUT CONTROLLER

*Fig.6*

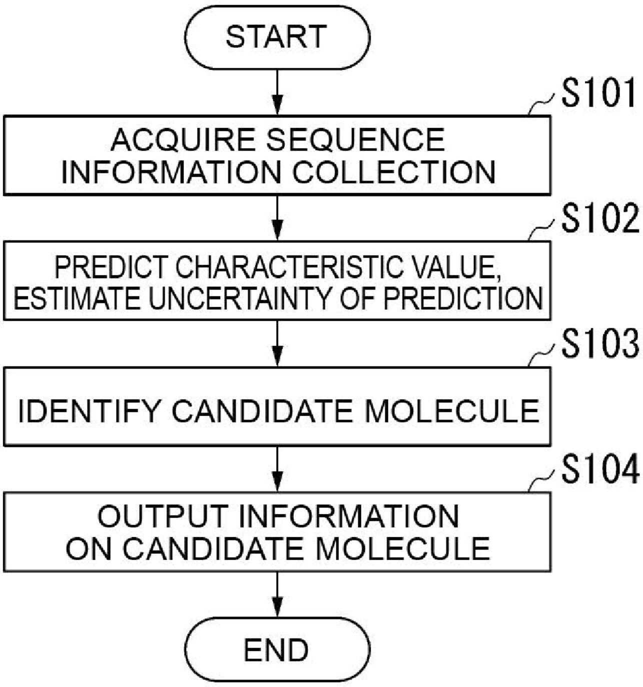
START
S101
ACQUIRE SEQUENCE INFORMATION COLLECTION
S102
PREDICT CHARACTERISTIC VALUE, ESTIMATE UNCERTAINTY OF PREDICTION
S103
IDENTIFY CANDIDATE MOLECULE
S104
OUTPUT INFORMATION ON CANDIDATE MOLECULE
END

Fig.7

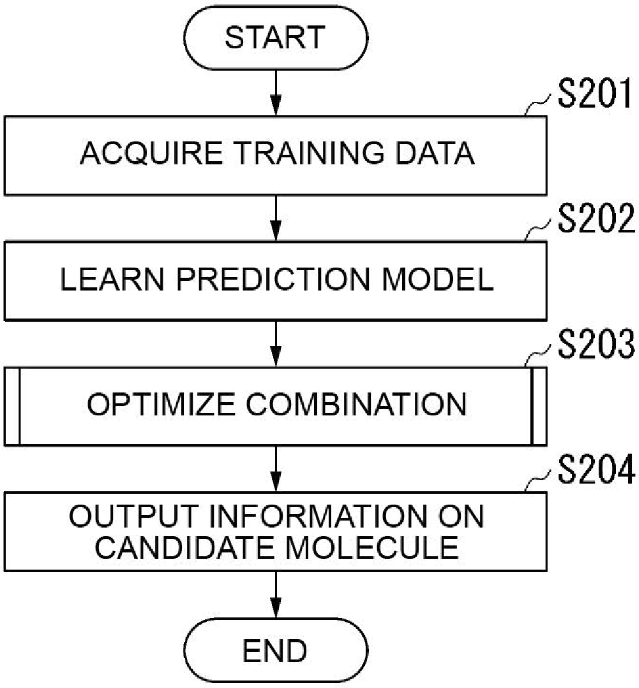
START
S201
ACQUIRE TRAINING DATA
S202
LEARN PREDICTION MODEL
S203
OPTIMIZE COMBINATION
S204
OUTPUT INFORMATION ON CANDIDATE MOLECULE
END

*Fig.8*

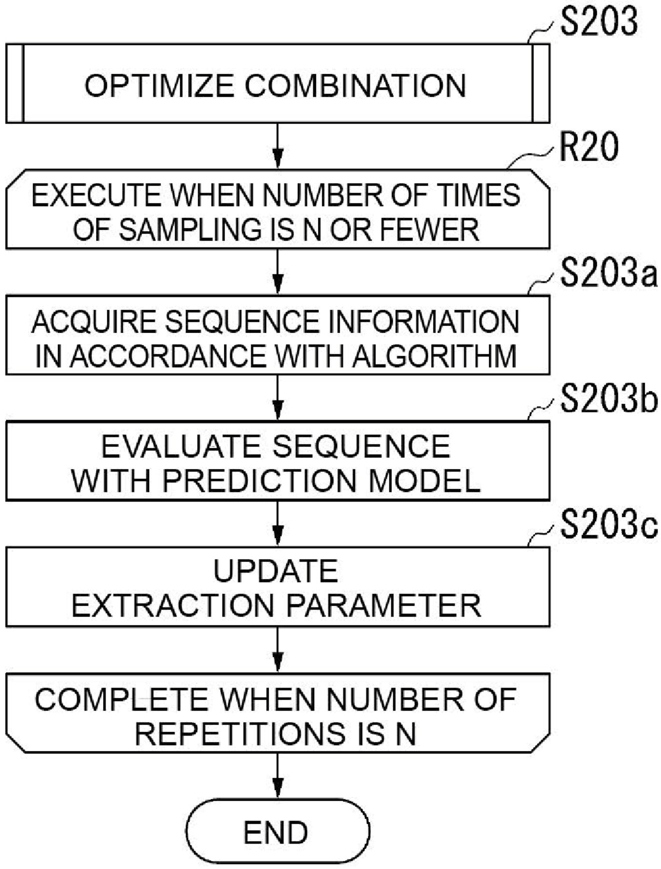
S203
OPTIMIZE COMBINATION
R20
EXECUTE WHEN NUMBER OF TIMES OF SAMPLING IS N OR FEWER
S203a
ACQUIRE SEQUENCE INFORMATION IN ACCORDANCE WITH ALGORITHM
S203b
EVALUATE SEQUENCE WITH PREDICTION MODEL
S203c
UPDATE EXTRACTION PARAMETER
COMPLETE WHEN NUMBER OF REPETITIONS IS N
END

Fig.9

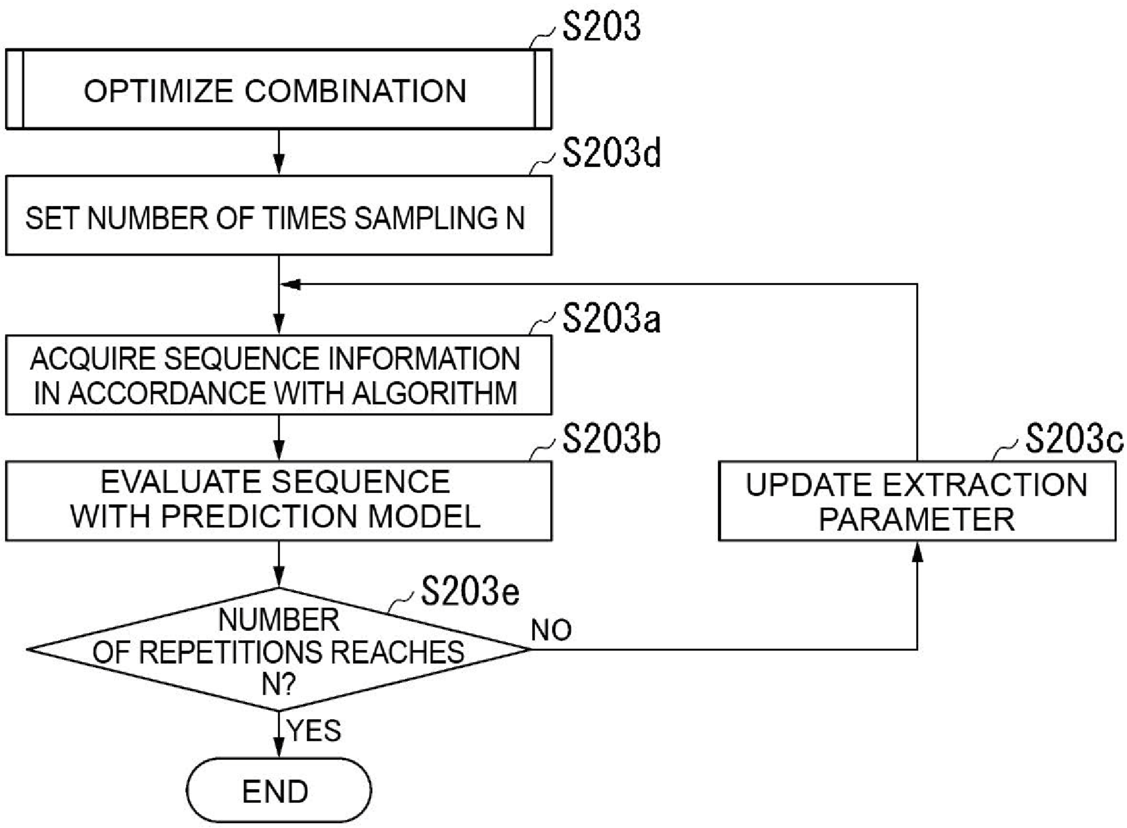
S203
OPTIMIZE COMBINATION
S203d
SET NUMBER OF TIMES SAMPLING N
S203a
ACQUIRE SEQUENCE INFORMATION IN ACCORDANCE WITH ALGORITHM
S203b
EVALUATE SEQUENCE WITH PREDICTION MODEL
S203c
UPDATE EXTRACTION PARAMETER
S203e
NUMBER OF REPETITIONS REACHES N?
NO
YES
END

*Fig.10*

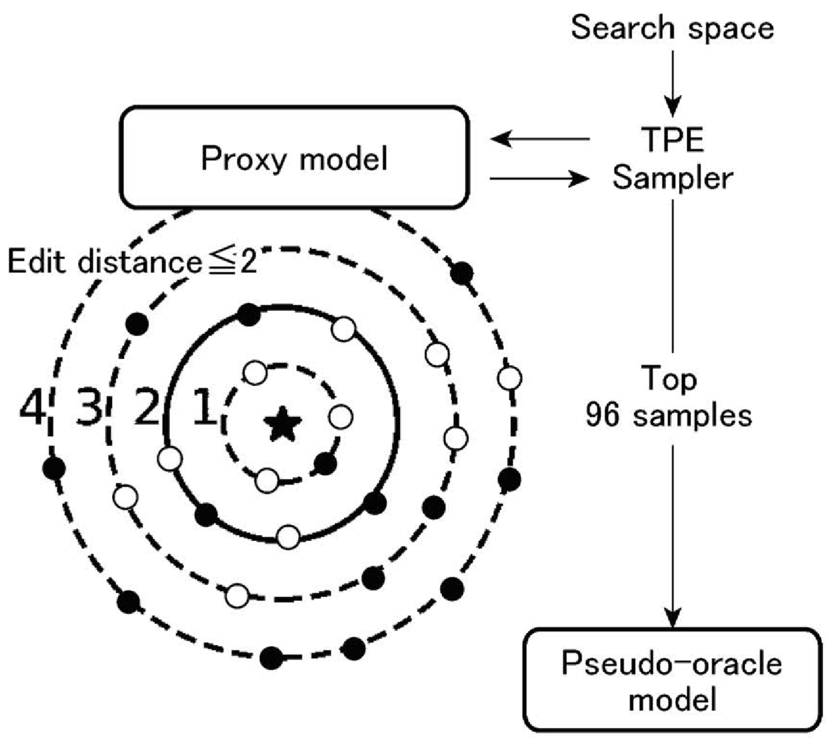
Search space
TPE
Sampler
Proxy model
Edit distance≦2
4
3
2
1
Top
96 samples
Pseudo-oracle
model

*Fig.11*

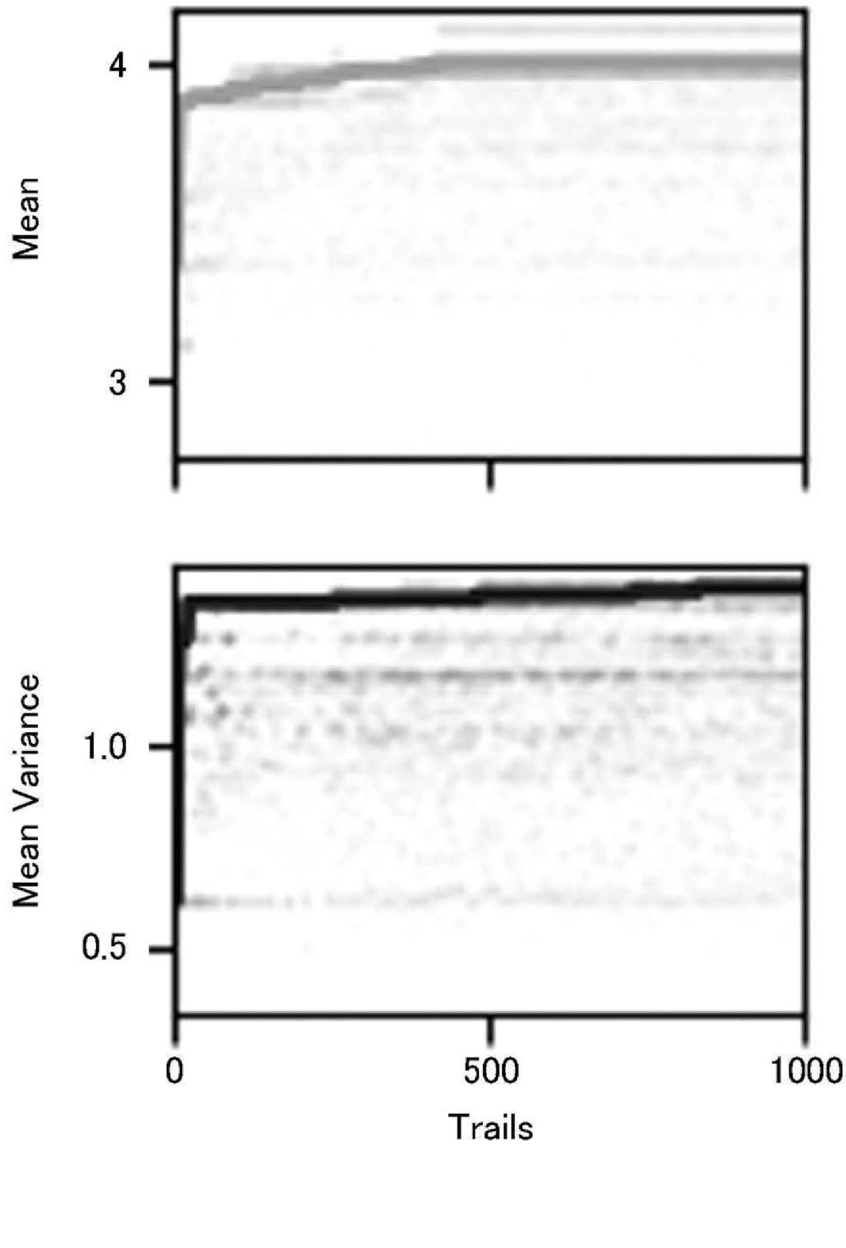

*Fig.12*

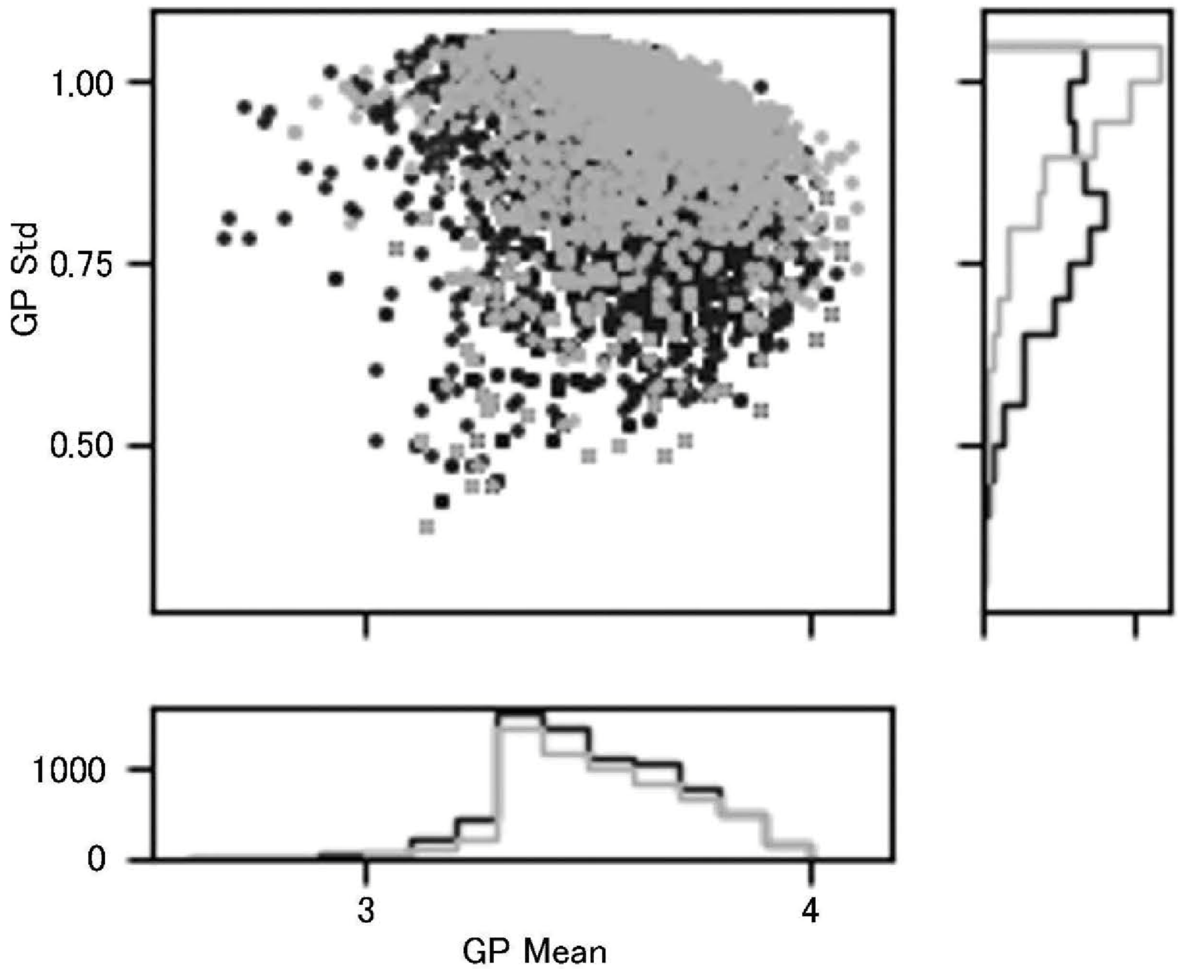

*Fig.13*

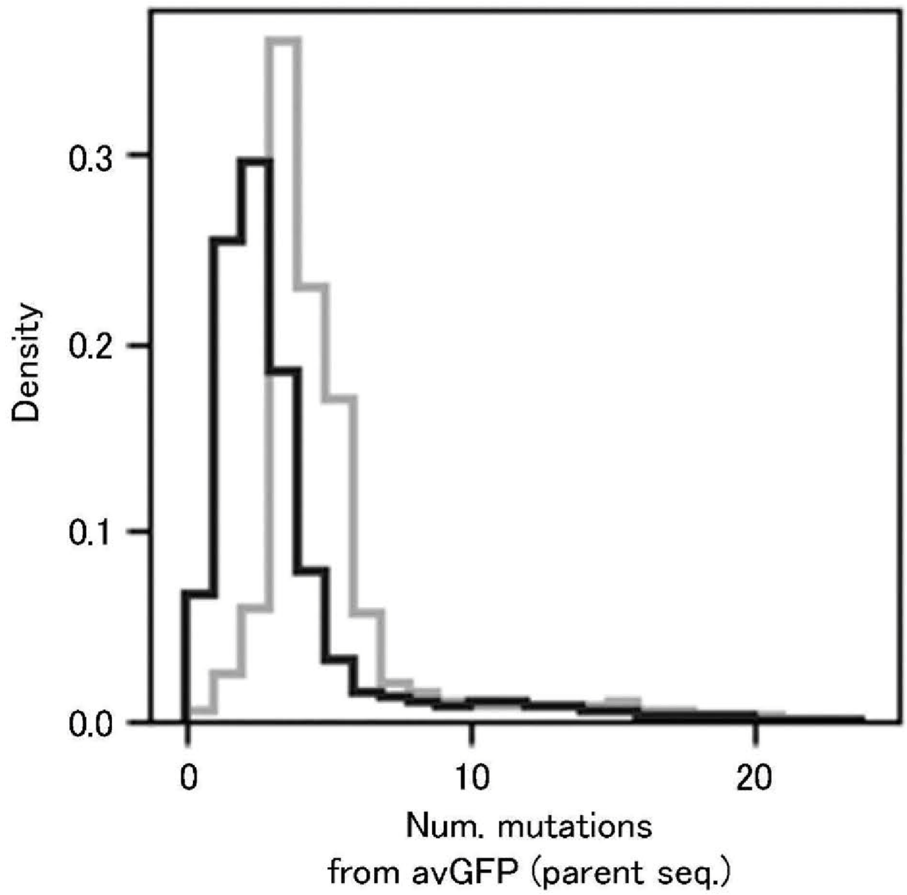
0.3
0.2
0.1
0.0
Density
0
10
20
Num. mutations
from avGFP (parent seq.)

*Fig.14*

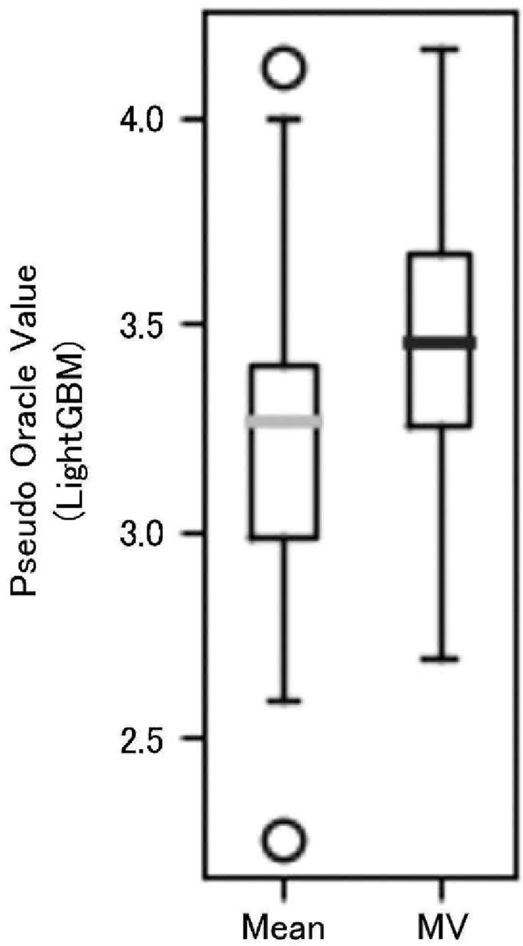

# *Fig.15*

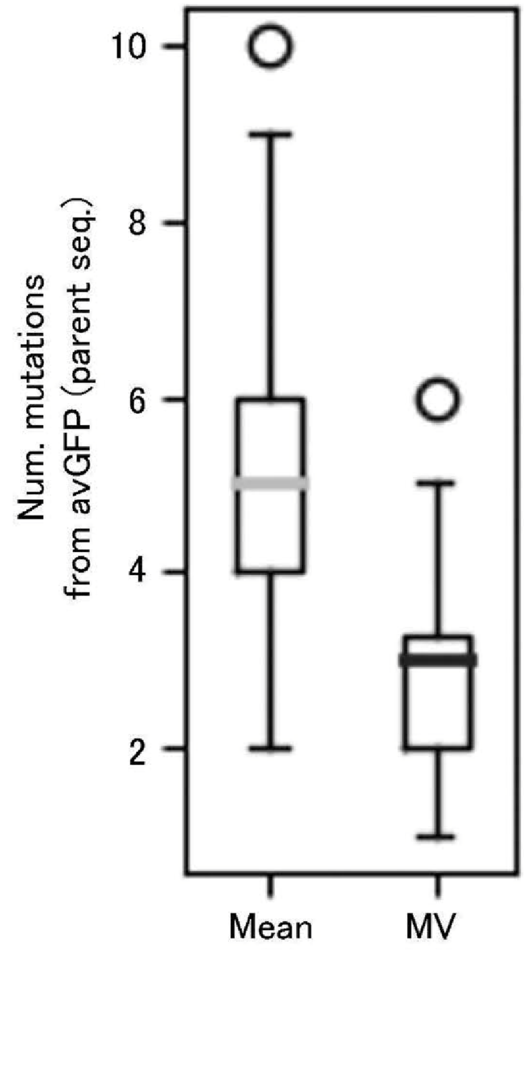

*Fig.16*

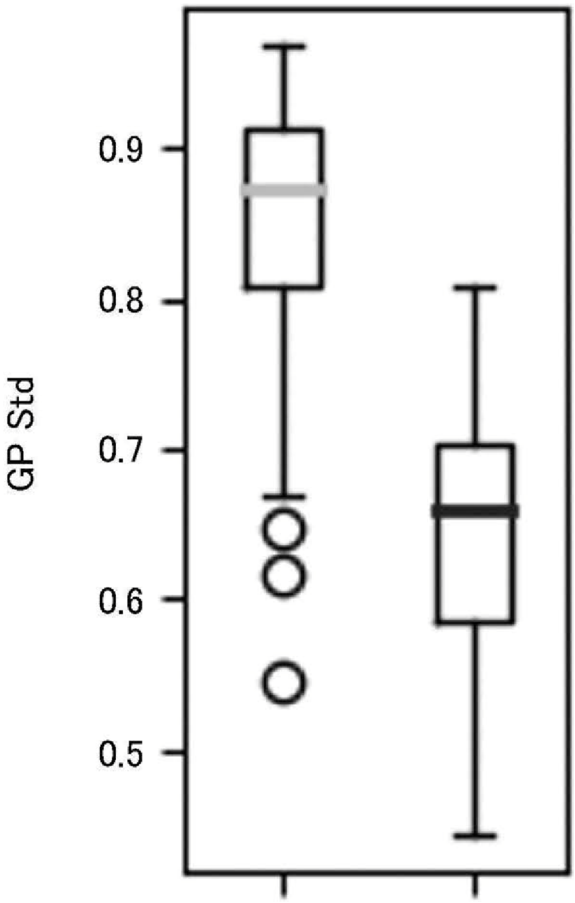

# Fig.17

| POSITION | CANDIDATE | POSITION | CANDIDATE | POSITION | CANDIDATE | POSITION | CANDIDATE | POSITION | CANDIDATE |
|---|---|---|---|---|---|---|---|---|---|
| 3 | AG | 72 | RL | 136 | LM | 174 | ST | 220 | LV |
| 18 | DE | 74 | HPT | 143 | NG | 177 | ILV | 230 | HY |
| 24 | QH | 104 | NCHIY | 144 | LY | 188 | CG | 231 | AG |
| 29 | CST | 105 | FY | 157 | GKV | 190 | GV | 233 | DY |
| 38 | NHSY | 109 | AS | 162 | AV | 197 | ND | 236 | NY |
| 41 | LM | 110 | QE | 163 | NT | 202 | IT | | |
| 43 | LM | 127 | AILT | 167 | RGH | 203 | QH | | |
| 61 | PST | 128 | DG | 170 | IL | 205 | AV | | |

Fig.18

| NAME | VALUE |
|---|---|
| NUMBER OF TIMES OF SAMPLING | 3000 |
| MULTIVARIABLE | NONE |

# Fig.19

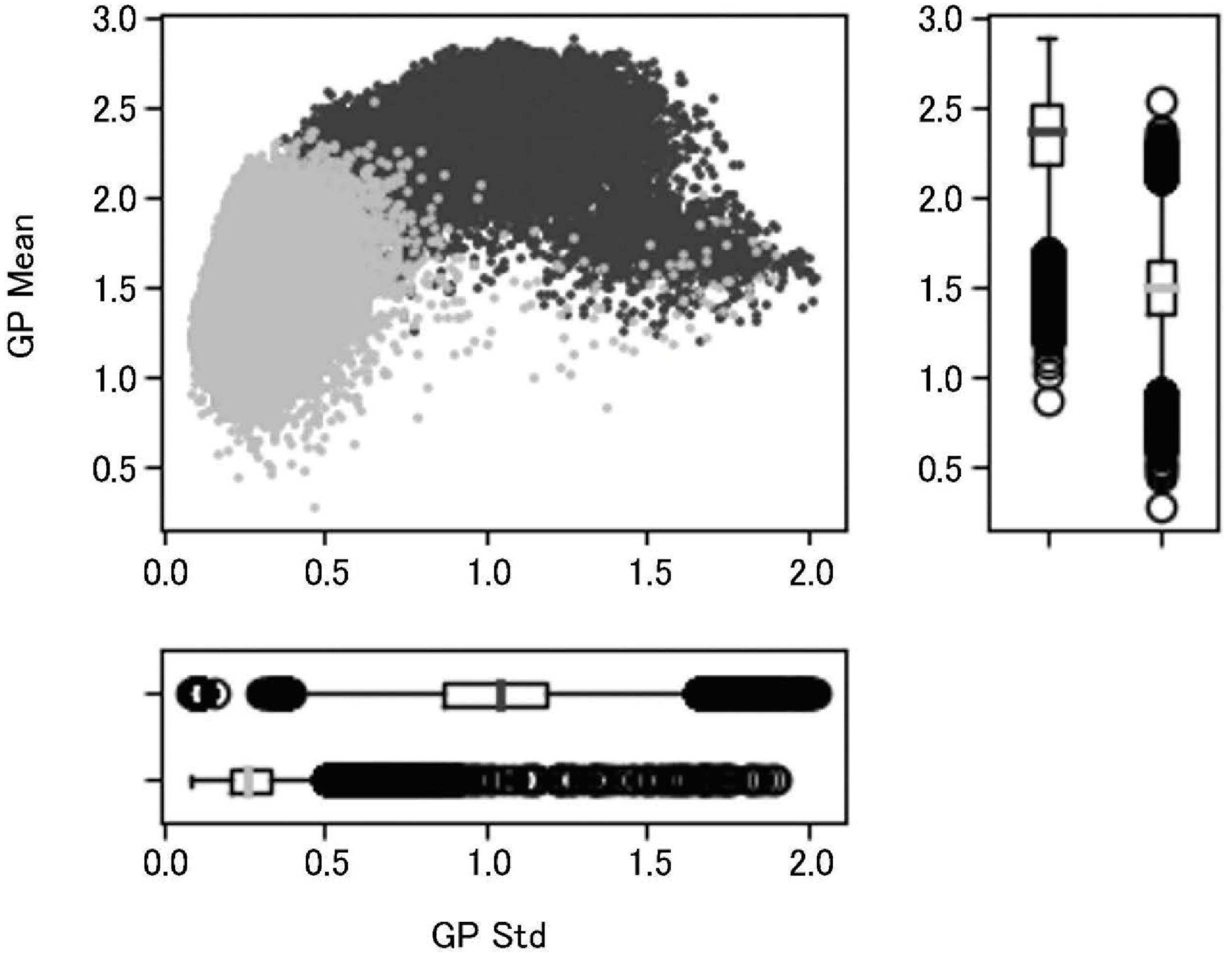

Fig.20

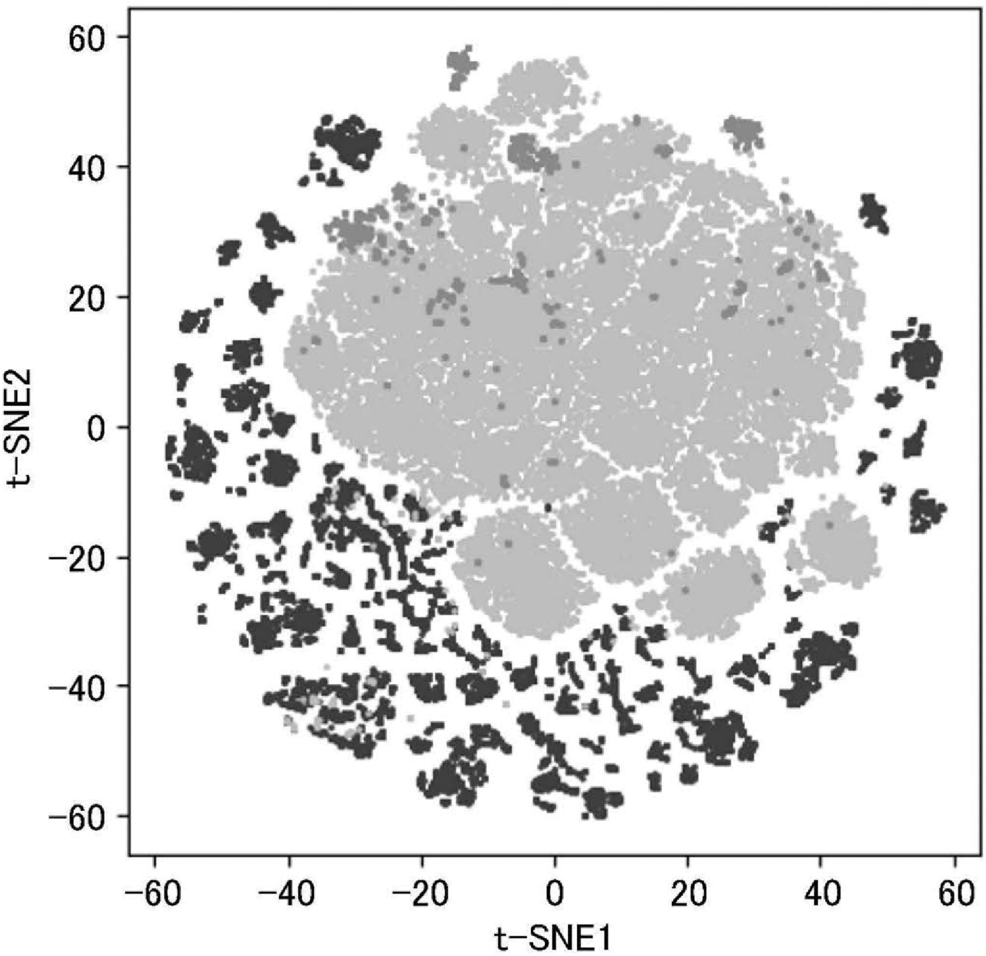

*Fig.21*

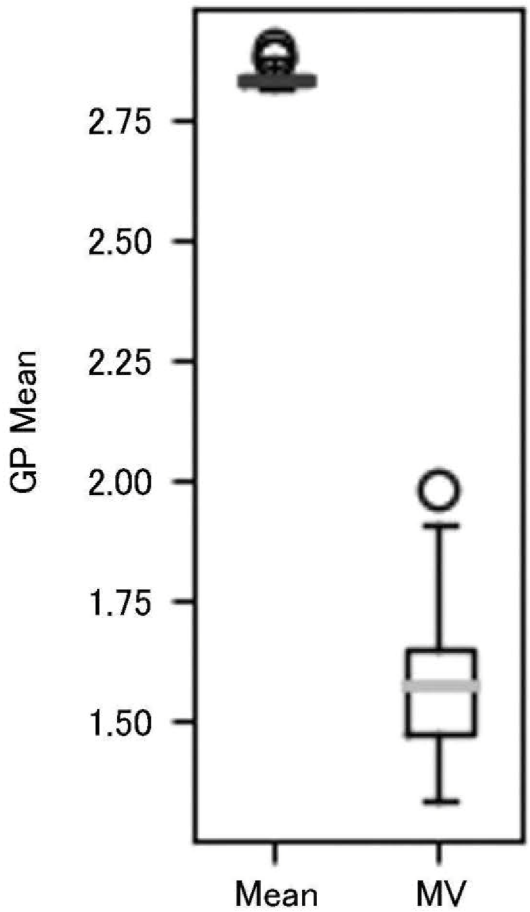

Fig.22

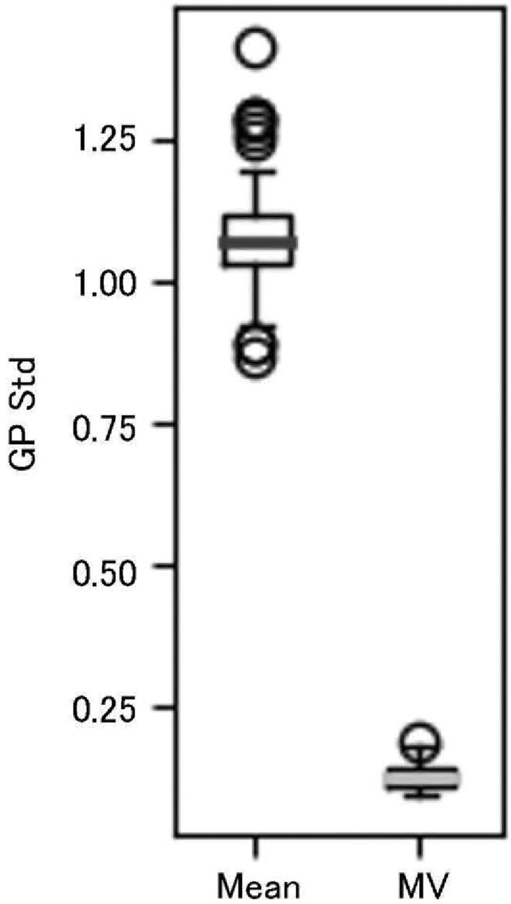

*Fig.23*

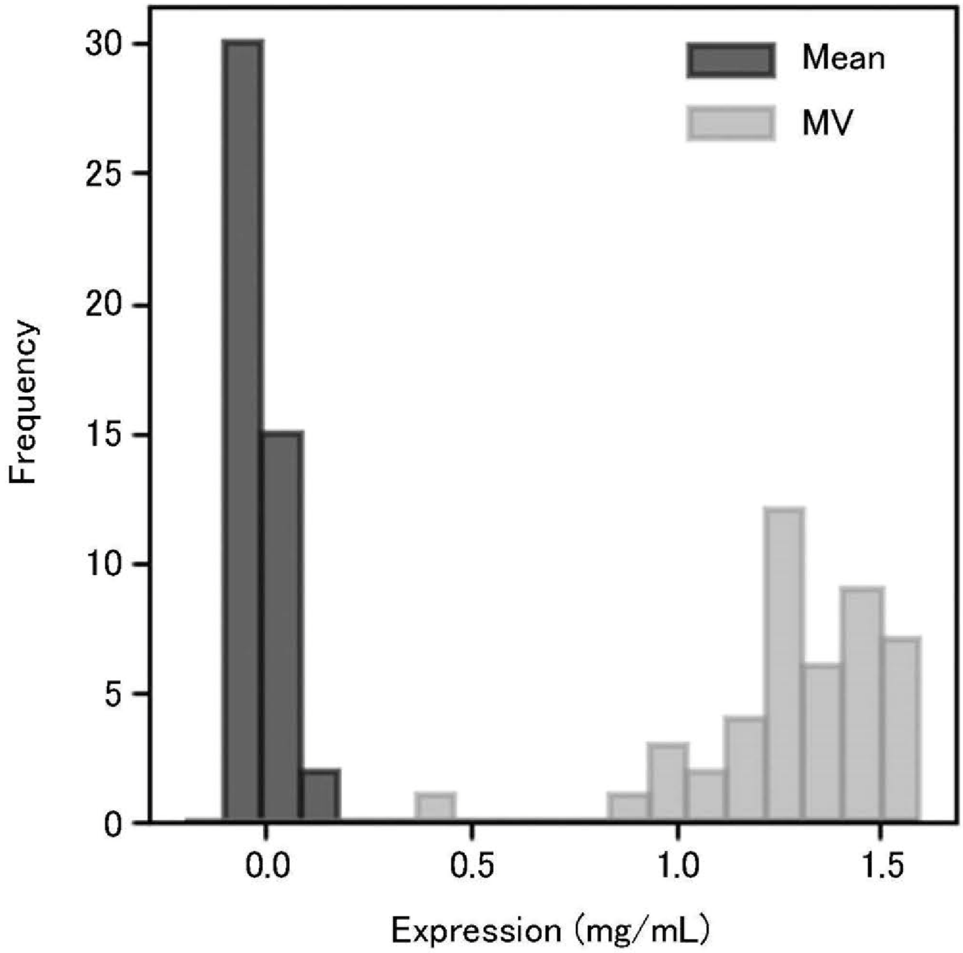

# Fig.24

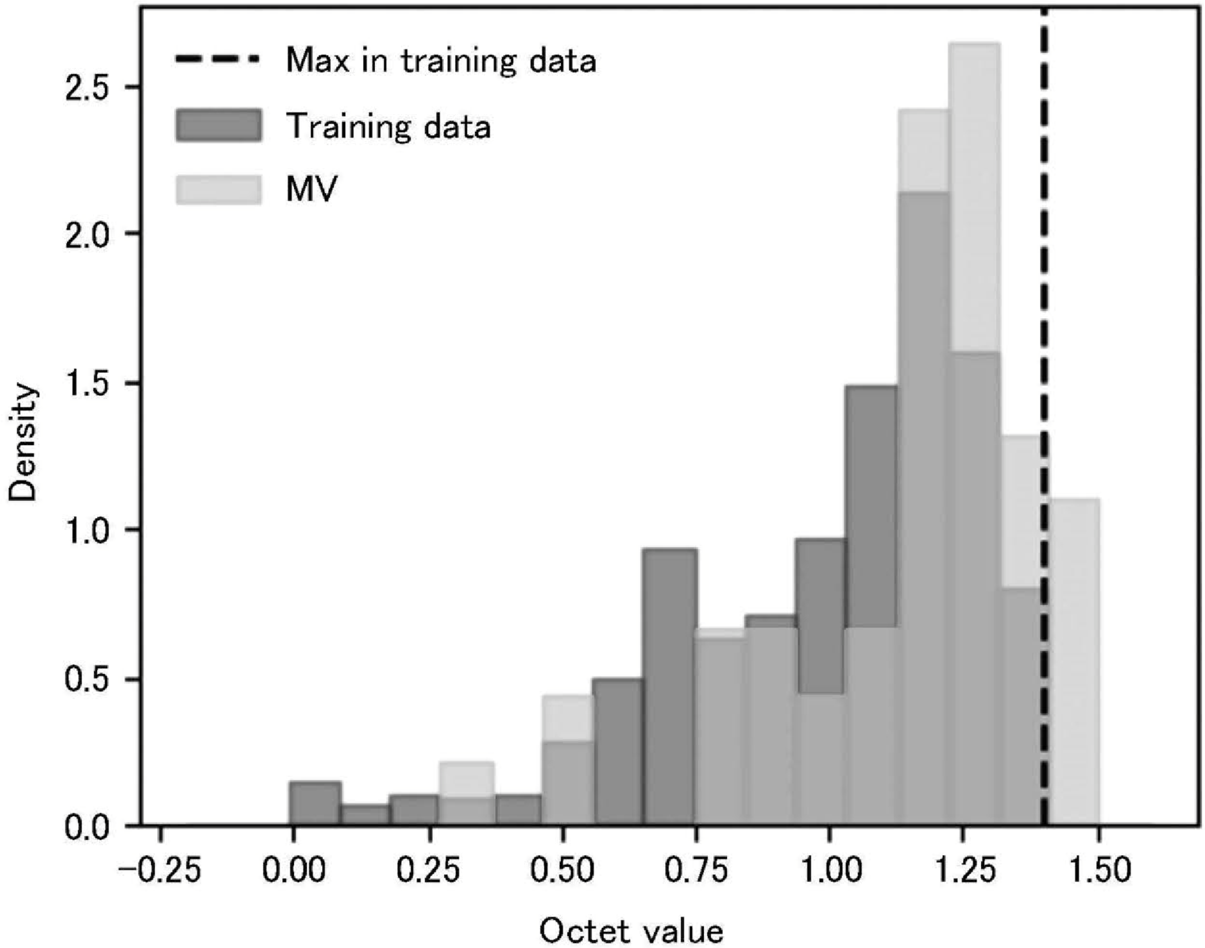
Max in training data
Training data
MV
Density
2.5
2.0
1.5
1.0
0.5
0.0
−0.25
0.00
0.25
0.50
0.75
1.00
1.25
1.50
Octet value

**INTERNATIONAL SEARCH REPORT**

International application No.
**PCT/JP2024/027412**

**A. CLASSIFICATION OF SUBJECT MATTER**

***G16C 20/50***(2019.01)i
FI: G16C20/50

According to International Patent Classification (IPC) or to both national classification and IPC

**B. FIELDS SEARCHED**

Minimum documentation searched (classification system followed by classification symbols)

G16C20/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2008-174503 A (NEC CORPORATION) 31 July 2008 (2008-07-31) entire text, all drawings | 1-20 |
| A | JP 2022-150078 A (FUJITSU LIMITED) 07 October 2022 (2022-10-07) entire text, all drawings | 1-20 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2024** | **03 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/027412**

| Patent document cited in search report | Publication date (day/month/year) | Patent family member(s) | Publication date (day/month/year) |
| --- | --- | --- | --- |
| JP 2008-174503 A | 31 July 2008 | (Family: none) | |
| JP 2022-150078 A | 07 October 2022 | US 2022/0310211 A1 | |
| | | EP 4071764 A1 | |
| | | CN 115132292 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018132752 A
- WO 2004044011 A
- WO 2005040229 A
- WO 2002032925 A
- WO 1995001937 A
- WO 2002020565 A
- WO 2003029462 A
- WO 2008016854 A
- US 4816567 A
- US 5648237 A
- US 5789199 A
- US 5840523 A
- WO 2020246617 A


### Non-patent literature cited in the description


- **KUNKEL et al.** *Proc. Natl. Acad. Sci. USA*, 1985, vol. 82, 488-492
- *Annu. Rev. Biophys. Biomol. Struct*, 2006, vol. 35, 225-249
- *Proc. Natl. Acad. Sci. U.S.A.*, 2003, vol. 100 (11), 6353-6357
- **KINDT et al.** Kuby Immunology. W.H. Freeman and Co., 2007, 91
- **PORTOLANO et al.** *J. Immunol.*, 1993, vol. 150, 880-887
- **CLARKSON et al.** *Nature*, 1991, vol. 352, 624-628
- **CHARLTON**. Methods in Molecular Biology. Humana Press, 2003, vol. 248, 245-254
- **Q. ZHU** ; **V. Y. F. TAN**. Thompson Sampling Algorithms for Mean-Variance Bandits. *ICML*, 2020
- **S. TAKEMORI**. Distributionally-Aware Kernelized Bandit Problems for Risk Aversion. *ICML*, 2022